# EUROPEAN PATENT APPLICATION

(11) **EP 4 752 144 A1**
(43) Date of publication of application: **03.06.2026**
(21) Application number: 24844955.5
(22) Date of filing: 19.07.2024
(51) Int. Cl.: C07D 405/04, H10K 50/15

(54) **ARYLAMINE COMPOUND, ORGANIC ELECTROLUMINESCENT ELEMENT, AND ELECTRONIC DEVICE**

(30) Priority: 26.07.2023 KR 20230097756
(71) Applicant: Hodogaya Chemical Co., Ltd., Tokyo, 105-0021 (JP)
(72) Inventor: KASE, Kouki, Tokyo (JP); LIM, Jae-Geon, Tokyo (JP); CHOI, Yeong-Tae, Tokyo (JP); SHIN, Heung-Seob, Tokyo (JP); IZUMIDA, Junichi, Tokyo (JP); HAYASHI, Shuichi, Tokyo (JP)
(74) Representative: Meissner Bolte Partnerschaft mbB
(86) International application number: PCT/IB2024/057008
(87) International publication number: WO 2025/022272

(57) **Abstract**

The objective of the present invention is to provide an organic compound having excellent properties as a material for a high-efficiency, high-durability organic EL element, such as exhibiting excellent hole injection and transport performance, having electron blocking ability and being highly stable in a thin film state, and to provide a high-efficiency, high-durability organic EL element using the compound. The arylamine compound of the present invention has excellent heat resistance and good hole transport ability. The organic EL element using the compound in the hole transport layer, electron blocking layer, light emitting layer and hole injection layer of the organic EL element exhibited good element properties.

## Description

### [TECHNICAL FIELD]

The present invention relates to a compound and an element suitable for an organic electroluminescent element (hereinafter referred to as "organic EL element"), which is a self-luminous electronic element preferable for various display devices, specifically to an arylamine compound and an organic EL element using the compound.

### [BACKGROUND ART]

Since organic EL elements are self-luminous elements, they are brighter than a liquid crystal device, have excellent visibility, and enable clear displays, and therefore, active research has been conducted on them.

In 1987, C. W. Tang and others at Eastman-Kodak made organic EL elements using organic materials practical by developing a layered structure element in which various roles were assigned to each material. They stacked a phosphor capable of transporting electrons and an organic material capable of transporting holes, and injected charges of both directions into the phosphor layer to emit light, thereby obtaining high luminance of 1000 cd/m² or more at a voltage of 10 V or less (see, e.g., Patent Document 1 and Patent Document 2).

To date, many improvements have been made for the practical use of organic EL elements, so that the roles of the laminated structure have been further subdivided, and high efficiency and durability is achieved by an electroluminescent element in which the anode, hole injection layer, hole transport layer, light emitting layer, electron transport layer, electron injection layer, and cathode are sequentially formed on a substrate (see, e.g., Non-Patent Document 1).

In addition, for the purpose of further improving luminous efficiency, the use of triplet excitons has been attempted, and the use of phosphorescent compounds has been examined (see, e.g., Non-Patent Document 2). In addition, elements using light emission by thermally activated delayed fluorescence (TADF) have also been developed, and in 2011, Adachi and others of Kyushu University realized an external quantum efficiency of 5.3% with an element using a thermally activated delayed fluorescent material (see, e.g., Non-Patent Document 3).

The light emitting layer may also be manufactured by doping a charge-transporting compound, generally called a host material, with a fluorescent compound, a phosphorescent compound, or a material that emits delayed fluorescence. As described in the above-mentioned non-patent document, the selection of organic materials in organic EL elements has a great influence on various characteristics such as efficiency and durability of the elements (see, e.g., Non-Patent Documents 1 to 3).

In organic EL elements, charges injected from both electrodes recombine in the light emitting layer so that light emission is obtained, but since it is important how to efficiently transfer the positive charges of holes and electrons to the light emitting layer, it is necessary to do it with an element having excellent carrier balance. Therefore, a material that has the characteristics of increasing the hole injection properties of supplying the holes injected from the anode to the light emitting layer and increasing the electron blocking properties of blocking the electrons injected from the cathode may be used, thereby increasing the probability that holes and electrons are recombined in the light emitting layer, and furthermore, the excitons generated within the light emitting layer may be confined, thereby obtaining high luminous efficiency. To this end, the role played by hole transport materials is important, and hole transport materials with high hole injection properties, high hole mobility, high electron blocking properties, and further high durability against electrons are required.

In addition, the heat resistance and amorphous properties of the materials are also important regarding the lifespan of the element. In materials with low heat resistance, thermal decomposition occurs even at low temperatures due to the heat generated when the element is driven, causing the materials to deteriorate. In materials with low amorphous properties, crystallization of the thin film occurs even in a short period of time, causing the element to deteriorate. For that reason, the materials used are required to have high heat resistance and good amorphous properties.

Hole transport materials that have been used so far in organic EL elements include N,N'-diphenyl-N,N'-di(α-naphthyl)benzidine (NPD) and various aromatic amine derivatives (see, e.g., Patent Document 1 and Patent Document 2). However, although NPD has a good hole transport ability, since its glass transition point (Tg), which is an indicator of heat resistance, is low at 96°C, the element characteristics are deteriorated due to crystallization under high temperature conditions (see, e.g., Non-Patent Document 4).

In addition, there are compounds with excellent hole mobility of 10⁻³ cm²/Vs or more among the aromatic amine derivatives described in the above patent documents (see, e.g., Patent Document 1 and Patent Document 2), but since their electron blocking properties are insufficient, some of the electrons escape the light emitting layer, and no improvement in luminous efficiency can be expected. In order to further increase efficiency, materials with higher electron blocking properties, more stable thin films, and higher heat resistance have been required. In addition, there have been reports of highly durable aromatic amine derivatives (see, e.g., Patent Document 3), but they are used as charge transport materials used in electrophotographic photoreceptors, and there have been no examples of their use as organic EL elements.

To solve this problem, substituted carbazole structures and arylamine compounds have been proposed as compounds with improved properties such as heat resistance and hole injection properties (see, e.g., Patent Document 4 and Patent Document 5). Although element lifespan and luminous efficiency have been improved in elements using these compounds in the hole injection layer or the hole transport layer, they cannot be said to be sufficient yet, and additional low driving voltage, high luminous efficiency, and long element lifespan have been required.

### [Prior Art Documents]

### [Patent Documents]

(Patent Document 1) US Patent No. 5792557 Specification
(Patent Document 2) US Patent No. 5639914 Specification
(Patent Document 3) US Patent No. 7759030 Specification
(Patent Document 4) Japanese Patent Laid-Open No. 2009-076817
(Patent Document 5) Japanese Patent No. 6674892
(Patent Document 6) European Patent No. 2684932 Specification
(Patent Document 7) Korean Patent Laid-Open No. 10-2022-0134847 Specification
(Patent Document 8) Korean Patent Laid-Open No. 10-2022-0154631 Specification

### [Non-Patent Documents]

(Non-Patent Document 1) The Japanese Journal of Applied Physics 9th Lecture Preliminary Book, pages 55 to 61 (2001)
(Non-Patent Document 2) The Japanese Journal of Applied Physics 9th Lecture Preliminary Book, pages 23 to 31 (2001)
(Non-Patent Document 3) Appl. Phys. Let., 98, 083302 (2011)
(Non-Patent Document 4) Organic EL Forum 3rd Annual Meeting Preview, pages 13 to 14 (2006)
(Non-Patent Document 5) Chem. Rev., 116, 12564 to 12649 (2016)

### [DISCLOSURE]

### [TECHNICAL PROBLEM]

An object of the present disclosure is to provide as a material for an organic EL element with high efficiency and high durability, which (1) has excellent hole injection and transport performance, (2) has electron blocking ability, (3) has high stability in a thin film state, and (4) has excellent durability.

It is to provide an organic EL element that (1) has high luminous efficiency and power efficiency, (2) has low luminescencestart voltage and practical driving voltage, and (3) has a long lifespan by using the material of the present disclosure.

### [TECHNICAL SOLUTION]

In order to achieve the above objects, the present inventors focused on the fact that arylamine compounds have excellent hole injection and transport abilities, and thin film stability and durability, and pursued optimization of substitution positions of the carbazolyl group and substituents, thereby dramatically improving the properties of the material. Even in organic EL elements, the performance of luminous efficiency and power efficiency has been improved, the luminescence start voltage and practical driving voltage have been suppressed, and a longer lifespan exceeding the conventional lifespan has been realized. As a result, the present disclosure has been completed.

That is, the present disclosure provides the following arylamine compounds and an organic EL element and electronic device using them.
1) An arylamine compound represented by the general formula (1) below:

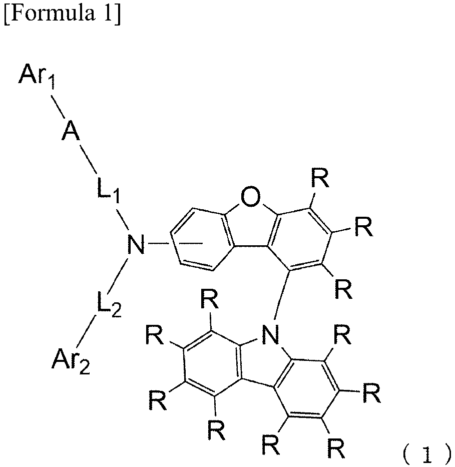

   wherein, in the formula, A indicates a substituted or unsubstituted naphthylene group,
   L₁ and L₂ indicate a single bond, a substituted or unsubstituted divalent aryl group, or
   a substituted or unsubstituted divalent heteroaryl group,
   R may be identical to or different from each other, and
   indicates a hydrogen atom, a deuterium atom, a substituted or unsubstituted aryl group, or
   a substituted or unsubstituted heteroaryl group, and
   Ar₁ and Ar₂ indicate a substituted or unsubstituted aryl group, or
   a substituted or unsubstituted heteroaryl group.
2) The arylamine compound described in 1) above, wherein the general formula (1) is represented by the general formula (1-1) or general formula (1-2) below: 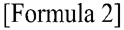 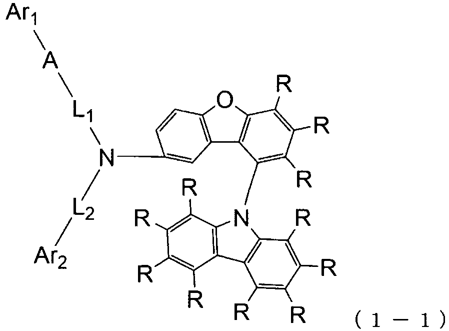 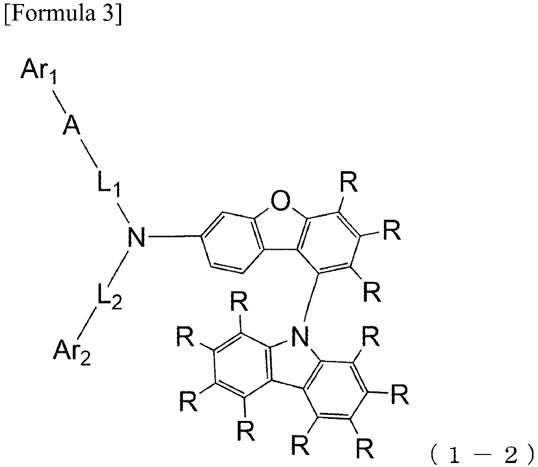 wherein, in the formulas, A, Ar₁, Ar₂, L₁, L₂ and R are as defined in the general formula (1).
3) The arylamine compound described in 2) above, wherein A in the general formula (1-1) or general formula (1-2) is represented by the general formula (2-1) to general formula (2-6) below:

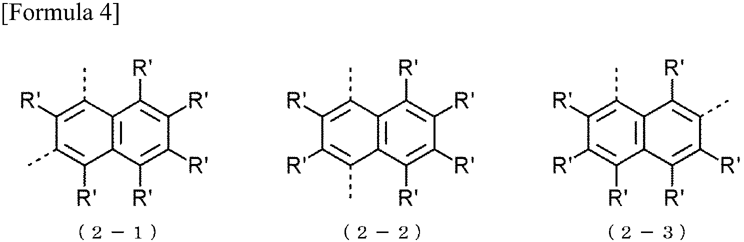

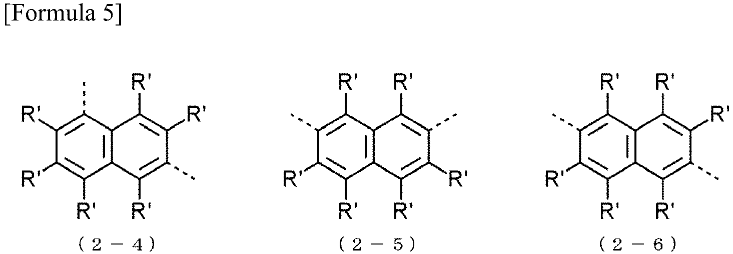

   wherein, in the formulas, a dashed line indicates a bonding position to Ar₁ or L₁, and
   R' may be identical to or different from each other, and
   indicates a hydrogen atom, a deuterium atom, an unsubstituted phenyl group, an unsubstituted biphenylyl group,
   an unsubstituted naphthyl group, or an unsubstituted phenanthryl group.
4) The arylamine compound described in 3), wherein L₁ and L₂ in the general formula (1-1) or (1-2) indicate
   a single bond, a substituted or unsubstituted divalent phenylene group, a substituted or unsubstituted divalent naphthylene group, or a substituted or unsubstituted divalent biphenylylene group.
5) The arylamine compound described in 3), wherein R in the general formula (1-1) or (1-2) indicates
   a hydrogen atom, a deuterium atom, a substituted or unsubstituted phenyl group, or a substituted or unsubstituted naphthyl group.
6) The arylamine compound described in 3), wherein Ar₁ in the general formula (1-1) or (1-2) indicates
   a substituted or unsubstituted phenyl group, a substituted or unsubstituted biphenylyl group,
   a substituted or unsubstituted naphthyl group, a substituted or unsubstituted dibenzofuranyl group, or
   a substituted or unsubstituted phenanthryl group.
7) The arylamine compound described in 6), wherein Ar₁ in the general formula (1-1) or (1-2) indicates
   an unsubstituted phenyl group, an unsubstituted biphenylyl group, an unsubstituted naphthyl group, an unsubstituted dibenzofuranyl group, or an unsubstituted phenanthryl group.
8) An organic EL element having a pair of electrodes and at least one organic layer sandwiched between them, wherein the organic layer contains the arylamine compound described in 1) or 2).
9) The organic EL element described in 8), wherein the organic layer is a hole transport layer, an electron blocking layer, a hole injection layer, or a light emitting layer.
10) An electronic device having a pair of electrodes and at least one organic layer sandwiched between them, wherein the organic layer contains the arylamine compound described in 1) or 2).

An 'aryl group' or 'heteroaryl group' in a 'substituted or unsubstituted aryl group' or 'substituted or unsubstituted heteroaryl group' represented by Ar₁, Ar₂ or R in the general formula (1) may include specifically
a phenyl group, a biphenylyl group, a terphenylyl group, a naphthyl group, an anthryl group, a phenanthryl group, an indenyl group, a pyrenyl group, a perylenyl group, a fluoranthenyl group, a triphenylenyl group, a pyridyl group, a pyrimidinyl group, a triazinyl group, a furyl group, a pyrrolyl group, a thienyl group, a quinolyl group, an isoquinolyl group, a benzofuranyl group, a benzothienyl group, an indolyl group, a carbazolyl group, a benzoxazolyl group, a benzothiazolyl group, a fluorenyl group, a spirobifluorenyl group, an azafluorenyl group, a diazafluorenyl group, an azaspirobifluorenyl group, a diazaspirobifluorenyl group, a quinoxalinyl group, a benzoimidazolyl group, a pyrazolyl group, a dibenzofuranyl group, a dibenzothienyl group, a naphthyridinyl group, a phenanthrolinyl group, an aclidinyl group, a carbolinyl group, etc., and may be further selected from an aryl group having 6 to 30 carbon atoms, or a heteroaryl group having 2 to 20 carbon atoms.

A 'substituent' in a 'substituted aryl group' or 'substituted heteroaryl group' represented by Ar₁, Ar₂ or R in the general formula (1) may include specifically
a deuterium atom, a cyano group, or a nitro group, a halogen atom such as a fluorine atom, a chlorine atom, a bromine atom, and an iodine atom, a silyl group such as a trimethylsilyl group and a triphenylsilyl group, a linear or branched alkyl group having 1 to 6 carbon atoms such as a methyl group, an ethyl group, and a propyl group, a linear or branched alkyloxy group having 1 to 6 carbon atoms such as a methyloxy group, an ethyloxy group, and a propyloxy group, an alkenyl group such as a vinyl group and an allyl group, an aryloxy group such as a phenyloxy group and a tolyloxy group, an arylalkyloxy group such as a benzyloxy group and a phenethyloxy group, an aromatic hydrocarbon group or condensed polycyclic aromatic group such as a phenyl group, a biphenylyl group, a terphenylyl group, a naphthyl group, an anthryl group, a phenanthryl group, a fluorenyl group, a spirofluorenyl group, an indenyl group, a pyrenyl group, a perylenyl group, a fluoranthenyl group, and a triphenylenyl group, and an aromatic heterocyclic group such as a pyridyl group, a thienyl group, a furyl group, a pyrrolyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzoimidazolyl group, a pyrazolyl group, a dibenzofuranyl group, a dibenzothienyl group, and a carbolinyl group. These substituents may further be substituted by the substituents exemplified above. Further, these substituents and the benzene ring substituted with the substituents, or a plurality of substituents substituted with the same benzene ring may be bonded to each other to form a ring by interposing a single bond, a substituted or unsubstituted methylene group, an oxygen atom, or a sulfur atom.

A 'divalent aryl group' or 'divalent heteroaryl group'
in a 'substituted or unsubstituted divalent aryl group' or
'substituted or unsubstituted divalent heteroaryl group'
represented by L₁ or L₂ in the general formula (1), may include
the same groups as those exemplified as the 'aryl group' or 'heteroaryl group' represented by Ar₁, Ar₂ or R in the general formula (1), and a group obtained by removing one hydrogen atom from such a group may be included as a divalent group.

A 'substituent' in a 'divalent aryl group having a substituent' or 'divalent heteroaryl group having a substituent' represented by L₁ or L₂ in the general formula (1) may include the same groups as those exemplified as the 'substituent' in the 'substituted aryl group' or 'substituted heteroaryl group' indicated by Ar₁, Ar₂ or R in the general formula (1), and the aspects that can be taken may also include the same ones.

A 'substituent' in a 'naphthylene group having a substituent' represented by A in the general formula (1) may include the same groups as those exemplified as the 'substituent' in the 'substituted aryl group' or 'substituted heteroaryl group' represented by Ar₁, Ar₂ or R in the general formula (1), and the aspects that can be taken may also include the same ones.

L₁ in the general formula (1) is preferably a substituted or unsubstituted phenylene group or a substituted or unsubstituted [1,1']biphenylylene group, more preferably an unsubstituted phenylene group, and even more preferably an unsubstituted 1,4-phenylene group.

Ar₁ and Ar₂ in the general formula (1) are preferably a substituted or unsubstituted phenyl group, a substituted or unsubstituted biphenylyl group, or a substituted or unsubstituted naphthyl group, more preferably an unsubstituted phenyl group, a deuterium-substituted phenyl group, an unsubstituted biphenylyl group, or an unsubstituted naphthyl group, and even more preferably an unsubstituted phenyl group or a deuterium-substituted phenyl group.

A in the general formula (1) is preferably the general formula (2-1), (2-3), or (2-4).

R in the general formula (1) is preferably a hydrogen atom, a deuterium atom, an unsubstituted phenyl group, or a deuterium-substituted phenyl group.

R' in the general formulas (2-1) to (2-6) is preferably a hydrogen atom, a deuterium atom, or an unsubstituted phenyl group, and more preferably a hydrogen atom or an unsubstituted phenyl group.

The arylamine compound represented by the general formula (1), which is preferably used in the organic EL element of the present disclosure, is preferably used as a constituent material of a hole injection layer, hole transport layer, electron blocking layer, or light emitting layer of the organic EL element, and more preferably used as a constituent material of a hole transport layer or an electron blocking layer.

In addition, the arylamine compound represented by the general formula (1) of the present disclosure is preferably used as a constituent material of an organic layer in an electronic device comprising a pair of electrodes and at least one organic layer sandwiched between the electrodes.

### [ADVANTAGEOUS EFFECTS]

Compared to conventional hole transport materials, the arylamine compound represented by the general formula (1) of the present disclosure has characteristics such as (1) good hole injection properties, (2) high hole mobility, (3) excellent electron blocking ability, (4) high electron resistance, (5) stable existence in a thin film state, (6) excellent heat resistance, etc., and characteristics such as (1) high luminous efficiency, (2) low luminescence start voltage, (3) low practical driving voltage, (4) long lifespan, etc. are obtained by using the arylamine compound represented by the general formula (1) of the present disclosure in an organic EL element.

The arylamine compound represented by the general formula (1) of the present disclosure is excellent in hole injection/transport performance, and thin film stability and durability. As a result, the organic EL element having a hole injection layer and/or a hole transport layer manufactured using the same compound as a hole injection material and/or a hole transport material improves luminous efficiency by improving the hole transport efficiency to the light emitting layer and enables the durability of the element to be improved by lowering the driving voltage, thereby making it possible to obtain characteristics of high efficiency, low driving voltage, and long lifespan.

The arylamine compound represented by the general formula (1) of the present disclosure has excellent electron blocking ability, has high electron resistance, is stable even in a thin film state, and has the characteristics of confining excitons generated in the light emitting layer. As a result, since the probability of recombining holes and electrons is improved to suppress thermal deactivation in an organic EL element having an electron blocking layer manufactured using the same compound as an electron blocking material, the organic EL element has high luminous efficiency, and the driving voltage is reduced, thereby improving current resistance to improve maximum luminance.

The arylamine compound represented by the general formula (1) of the present disclosure has excellent hole transport properties and has a wide band gap. As a result, in an organic EL element having a light emitting layer manufactured using the same compound as a host material, the driving voltage is lowered, and the luminous efficiency is improved by forming the light emitting layer by supporting a fluorescent light emitter, a phosphorescent light emitter, or a delayed fluorescent light emitter, which is called a dopant.

Therefore, the arylamine compound represented by the general formula (1) of the present disclosure is useful as a constituent material for the hole injection layer, hole transport layer, electron blocking layer, or light emitting layer of an organic EL element, and can improve the luminous efficiency, driving voltage, and durability of a conventional organic EL element.

In addition, the arylamine compound represented by the general formula (1) of the present disclosure can be used not only in organic EL elements but also in the field of electronic devices such as electrophotographic photoreceptors, image sensors, photoelectric conversion elements, and solar cells.

### [DESCRIPTION OF DRAWINGS]

FIG. 1 is a drawing showing the structures of Compound (A-1) to (A-20) as arylamine compounds represented by the general formula (1).
FIG. 2 is a drawing showing the structures of Compound (A-21) to (A-44) as arylamine compounds represented by the general formula (1).
FIG. 3 is a drawing showing the structures of Compound (A-45) to (A-64) as arylamine compounds represented by the general formula (1).
FIG. 4 is a drawing showing the structures of Compound (A-65) to (A-88) as arylamine compounds represented by the general formula (1).
FIG. 5 is a drawing showing the structures of Compound (A-89) to (A-108) as arylamine compounds represented by the general formula (1).
FIG. 6 is a drawing showing the structures of Compound (A-109) to (A-128) as arylamine compounds represented by the general formula (1).
FIG. 7 is a drawing showing the structures of Compound (A-129) to (A-148) as arylamine compounds represented by the general formula (1).
FIG. 8 is a drawing showing the structures of Compound (A-149) to (A-164) as arylamine compounds represented by the general formula (1).
FIG. 9 is a drawing showing the structures of Compound (A-165) to (A-186) as arylamine compounds represented by the general formula (1).
FIG. 10 is a drawing showing the structures of Compound (A-187) to (A-210) as arylamine compounds represented by the general formula (1).
FIG. 11 is a drawing showing the structures of Compound (A-211) to (A-230) as arylamine compounds represented by the general formula (1).
FIG. 12 is a drawing showing the structures of Compound (A-231) to (A-236) as arylamine compounds represented by the general formula (1).
FIG. 13 is a drawing showing the structures of Compound (B-1) to (B-20) as arylamine compounds represented by the general formula (1).
FIG. 14 is a drawing showing the structures of Compound (B-21) to (B-40) as arylamine compounds represented by the general formula (1).
FIG. 15 is a drawing showing the structures of Compound (B-41) to (B-60) as arylamine compounds represented by the general formula (1).
FIG. 16 is a drawing showing the structures of Compound (B-61) to (B-80) as arylamine compounds represented by the general formula (1).
FIG. 17 is a drawing showing the structures of Compound (B-81) to (B-100) as arylamine compounds represented by the general formula (1).
FIG. 18 is a drawing showing the structures of Compound (B-101) to (B-120) as arylamine compounds represented by the general formula (1).
FIG. 19 is a drawing showing the structures of Compound (B-121) to (B-140) as arylamine compounds represented by the general formula (1).
FIG. 20 is a drawing showing the structures of Compound (B-141) to (B-161) as arylamine compounds represented by the general formula (1).
FIG. 21 is a drawing showing the structures of Compound (B-162) to (B-181) as arylamine compounds represented by the general formula (1).
FIG. 22 is a drawing showing the structures of Compound (B-182) to (B-201) as arylamine compounds represented by the general formula (1).
FIG. 23 is a drawing showing the structures of Compound (B-202) to (B-221) as arylamine compounds represented by the general formula (1).
FIG. 24 is a drawing showing the structures of Compound (B-222) to (B-241) as arylamine compounds represented by the general formula (1).
FIG. 25 is a drawing showing the structures of Compound (B-242) to (B-261) as arylamine compounds represented by the general formula (1).
FIG. 26 is a drawing showing the structures of Compound (B-262) to (B-267) as arylamine compounds represented by the general formula (1).
FIG. 27 is a drawing showing the organic EL element configurations of Examples 19 to 34 and Comparative Examples 1 to 2.

### [BEST MODE FOR CARRYING OUT THE INVENTION]

Although the arylamine compounds represented by the general formula (1) of the present disclosure are novel compounds, these compounds can be synthesized according to methods known per se (see, e.g., Non-Patent Document 5).

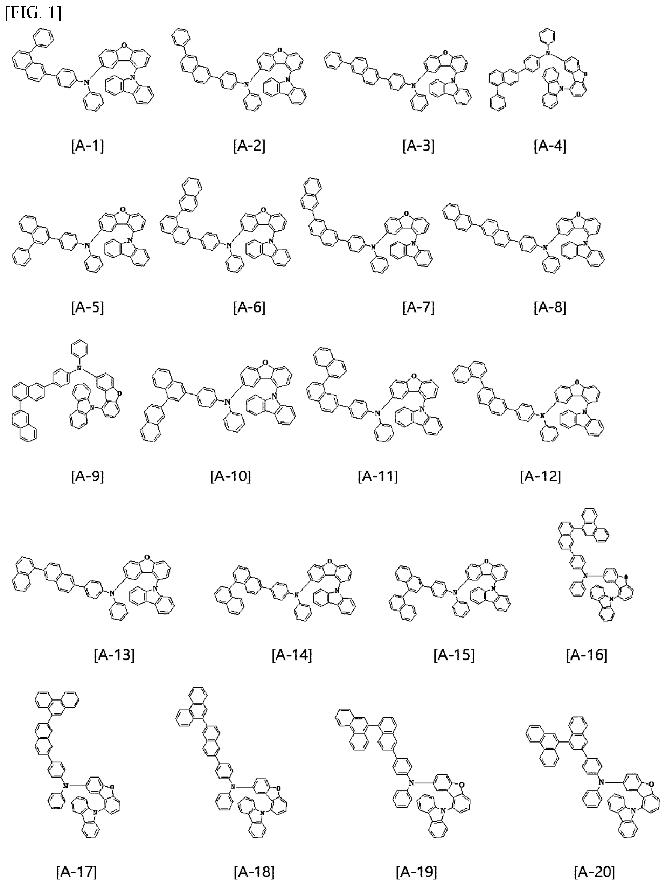

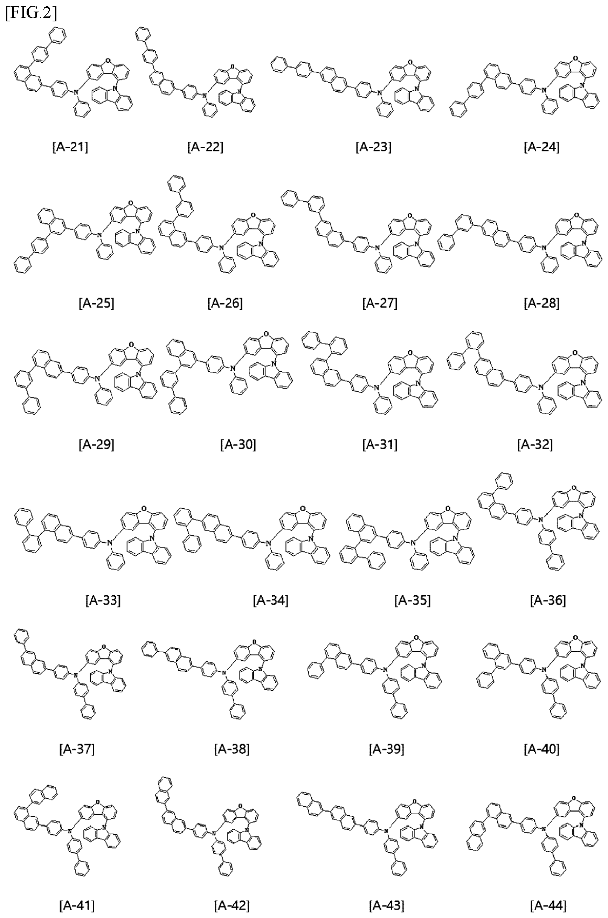

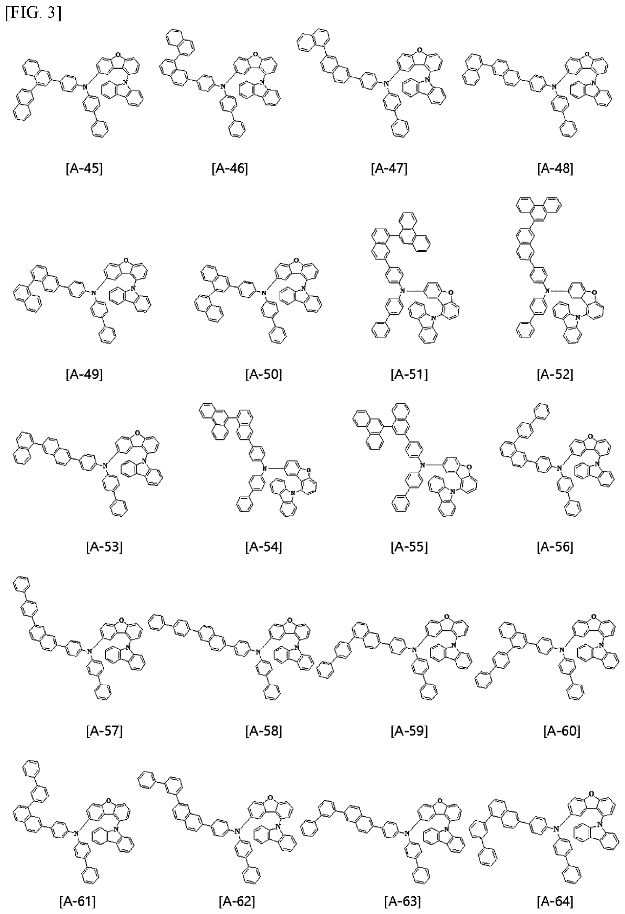

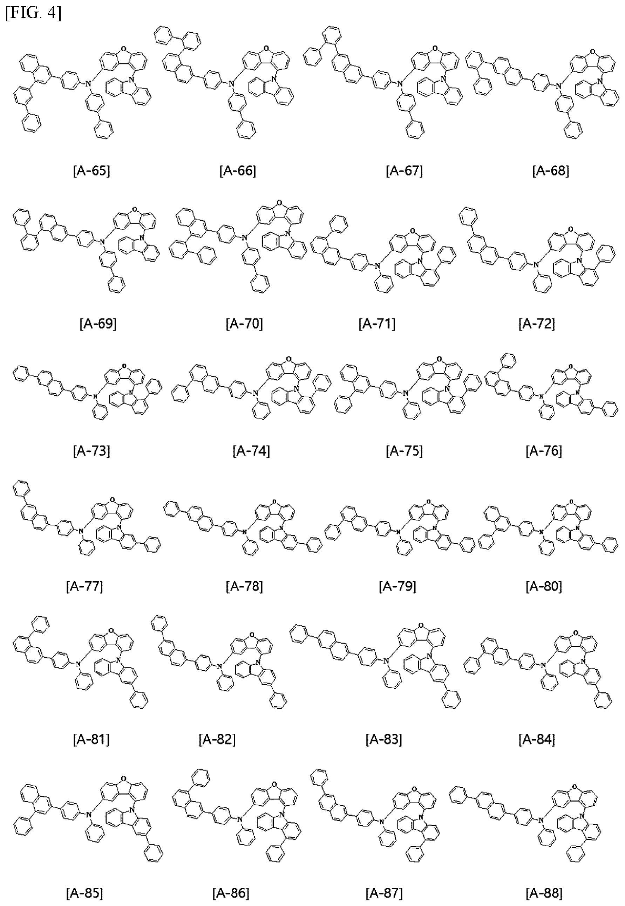

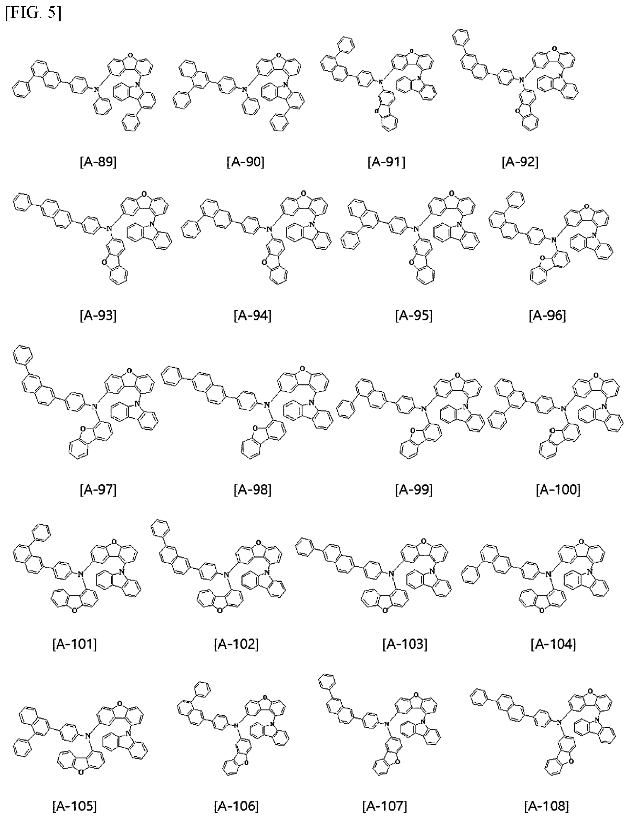

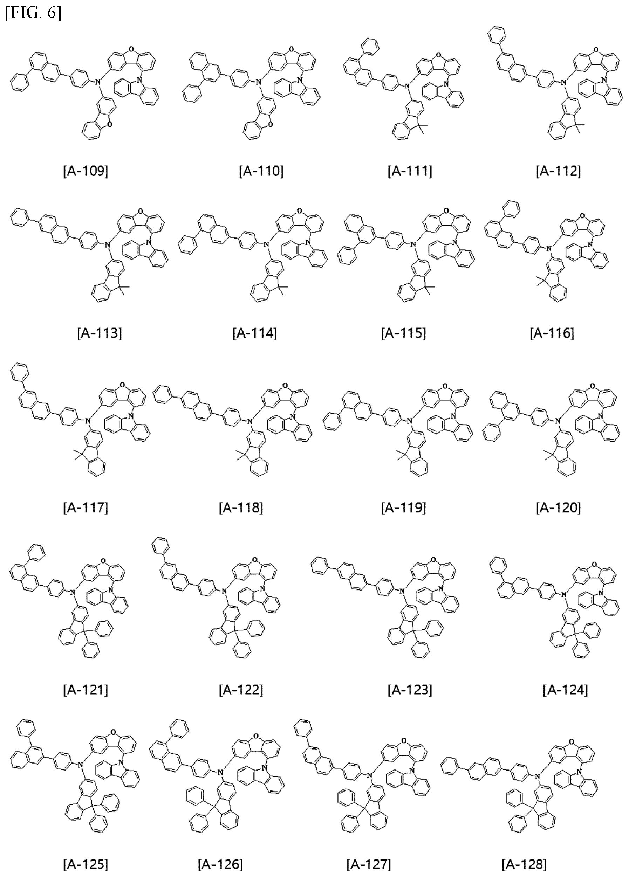

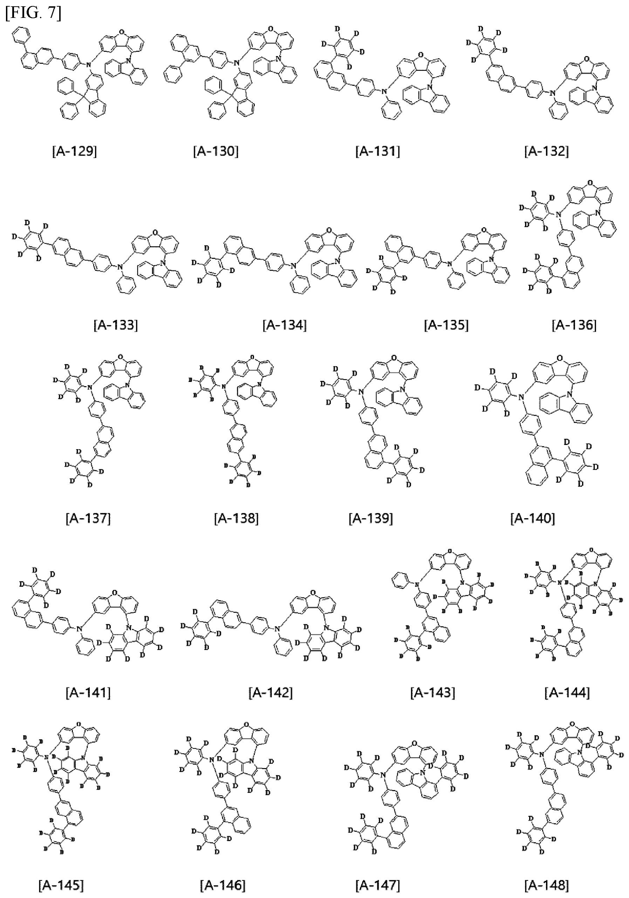

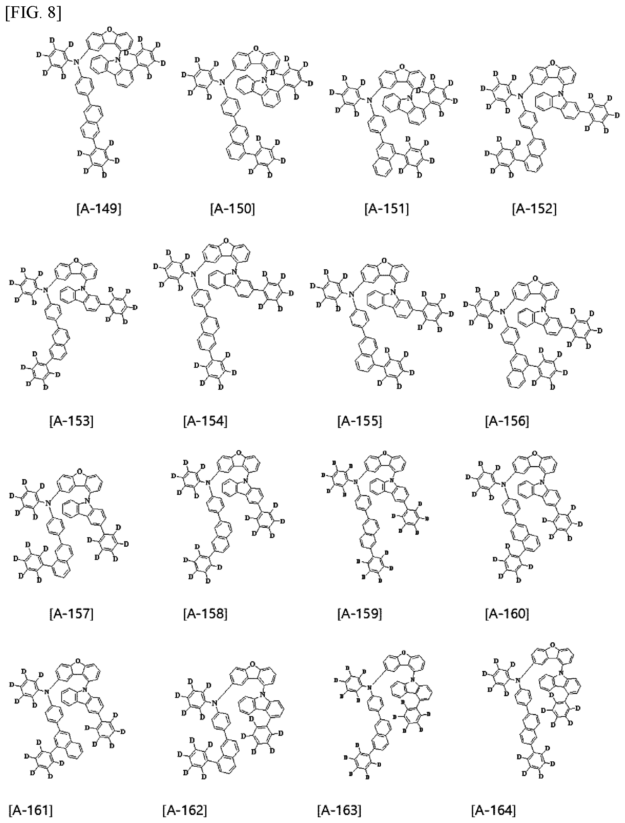

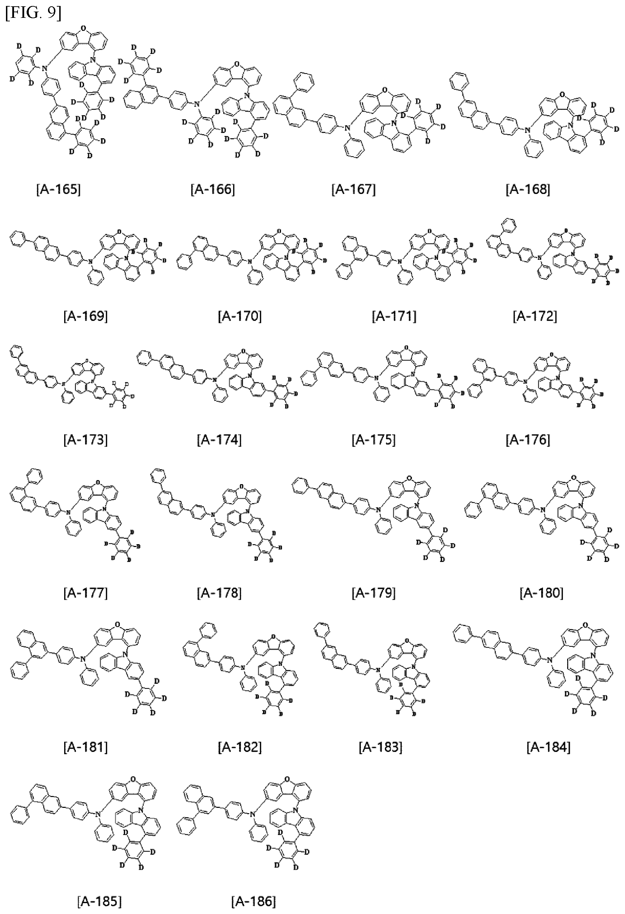

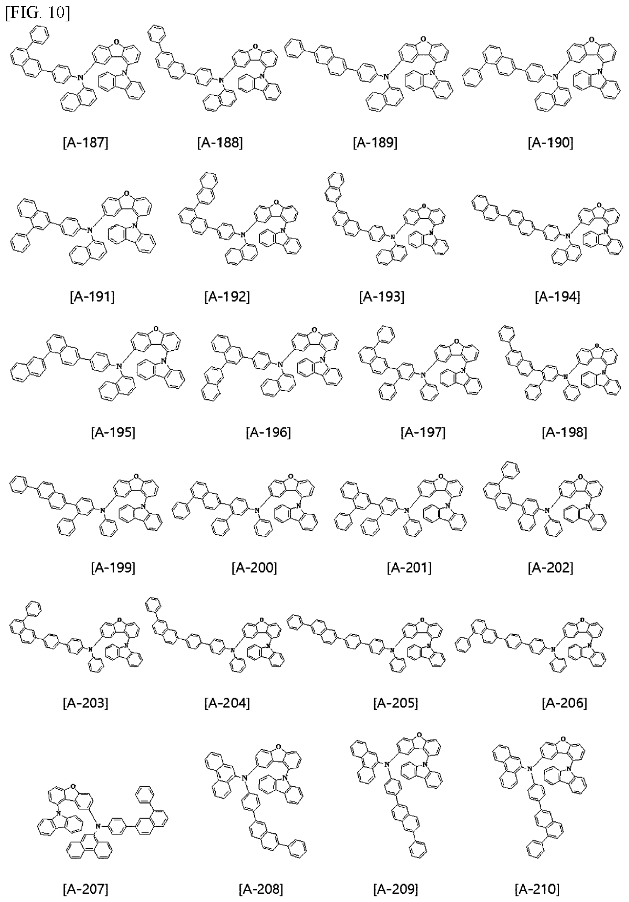

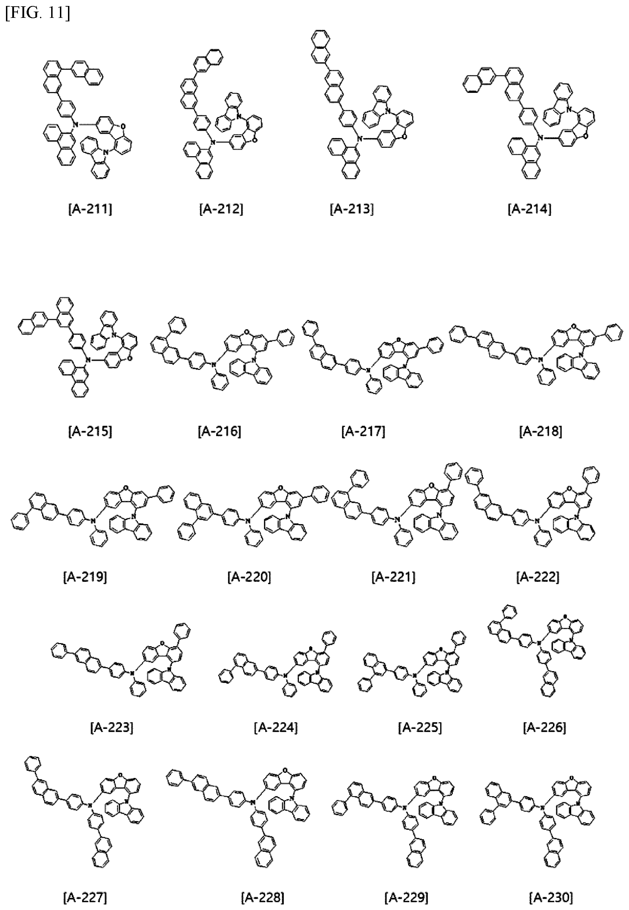

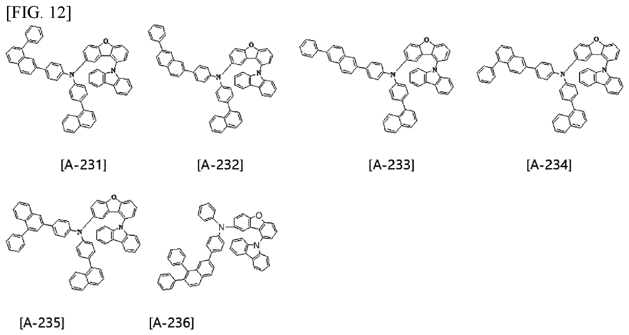

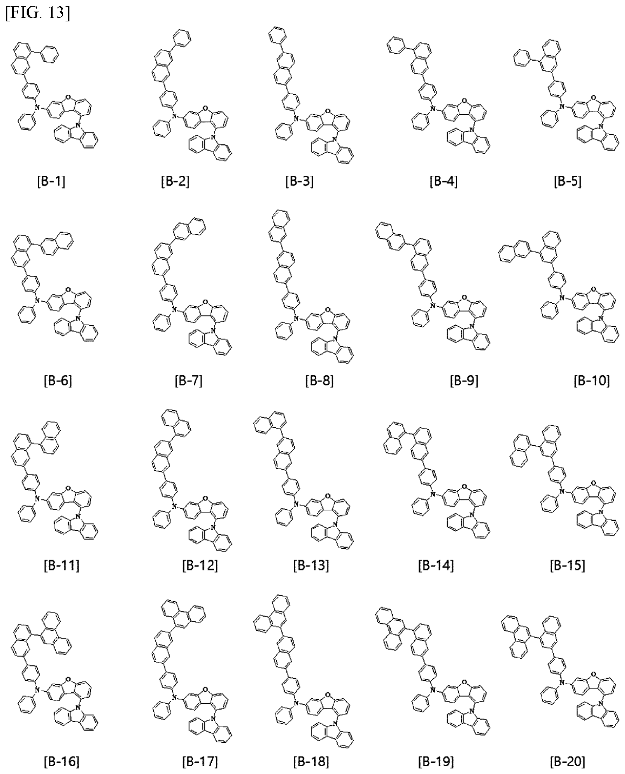

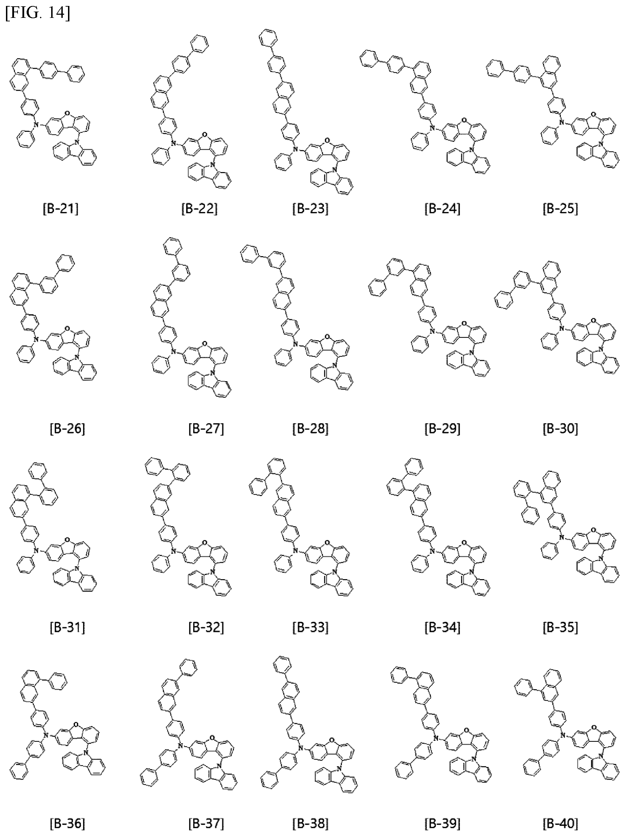

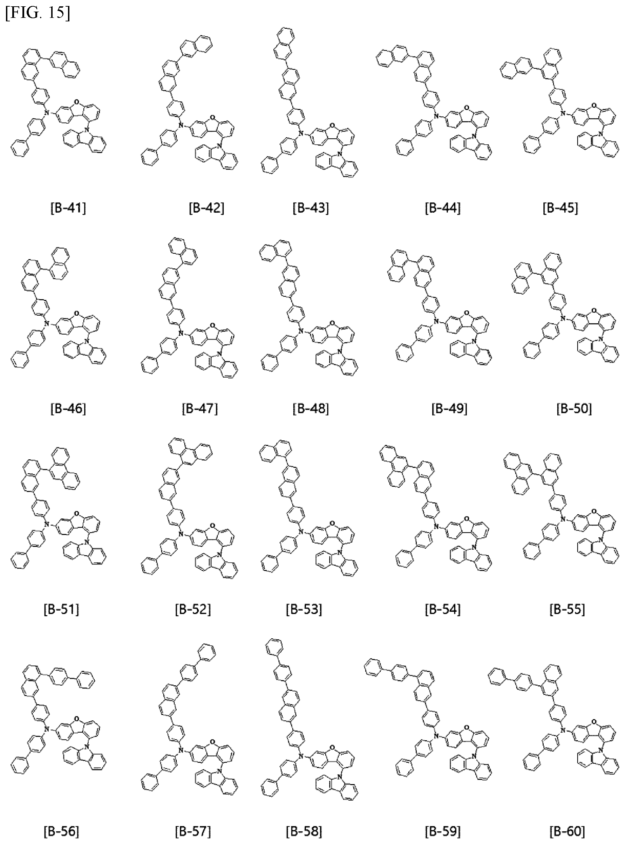

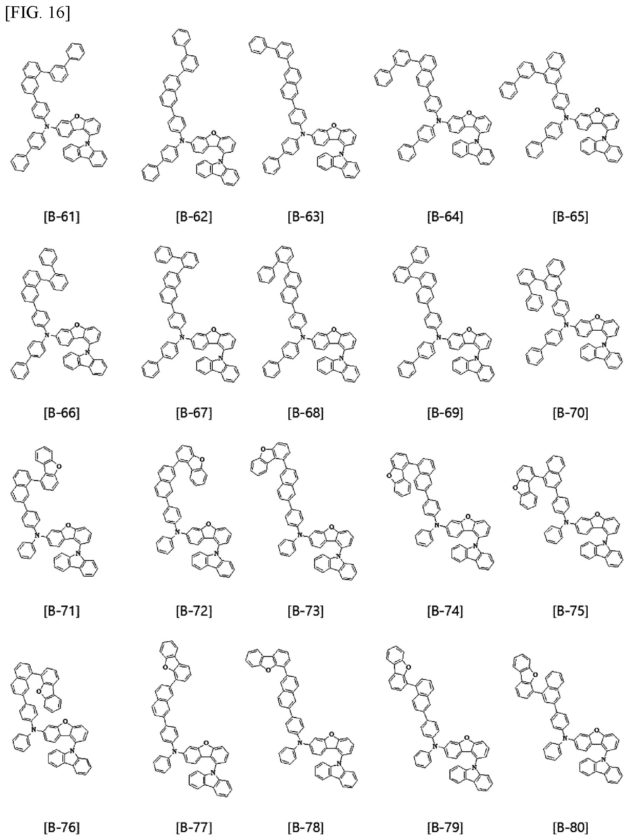

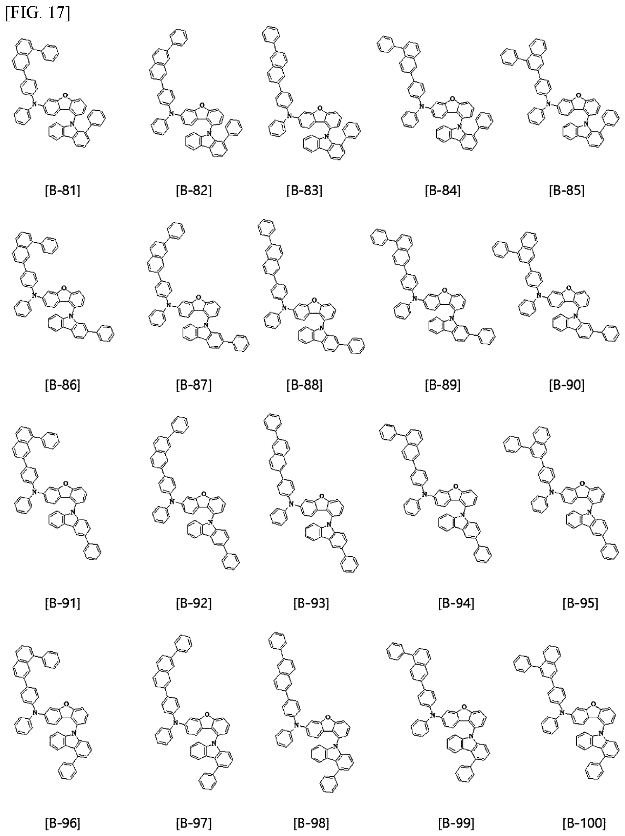

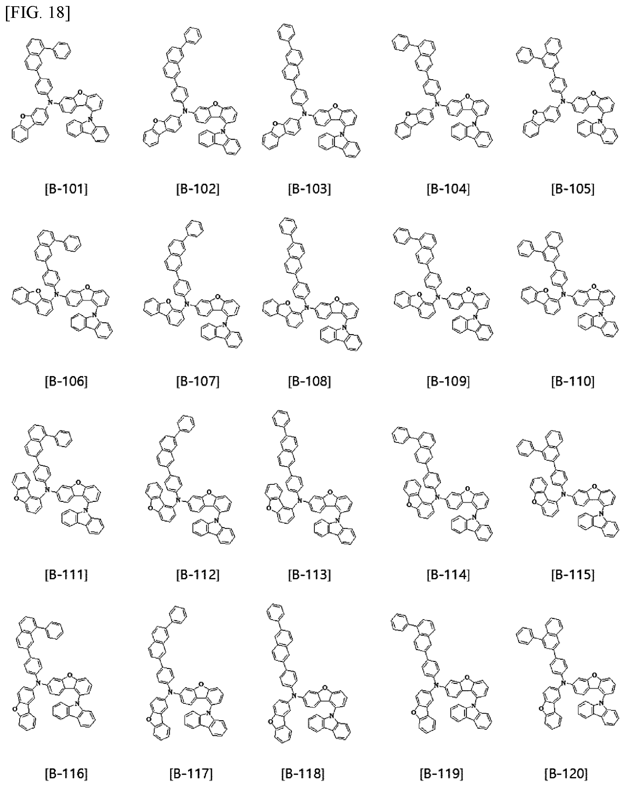

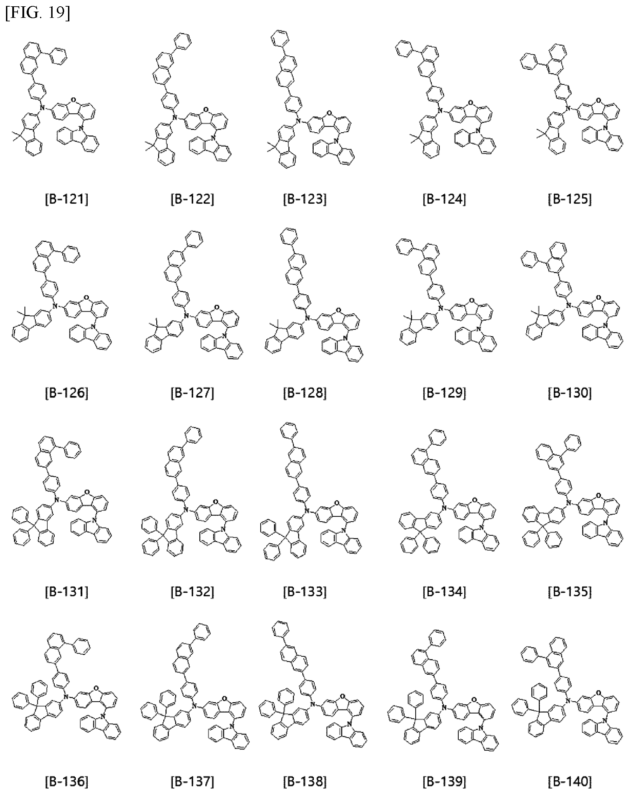

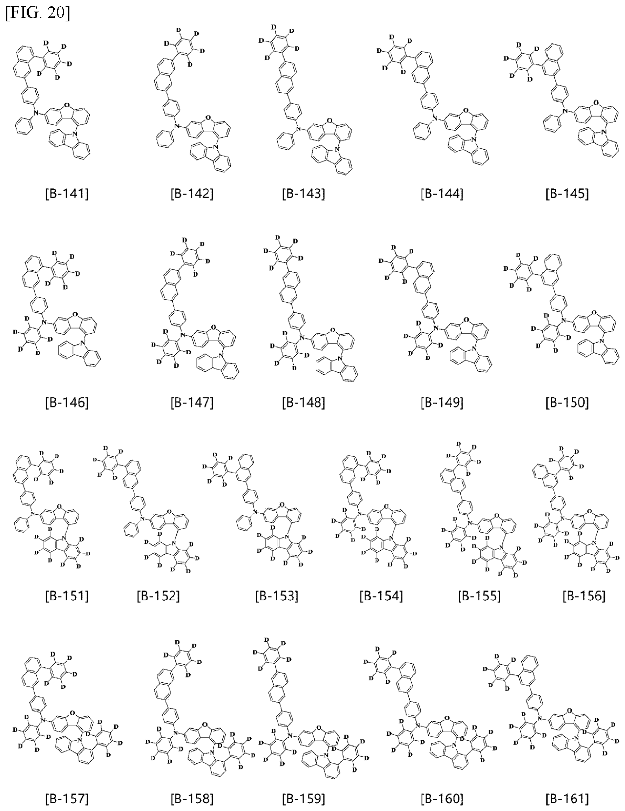

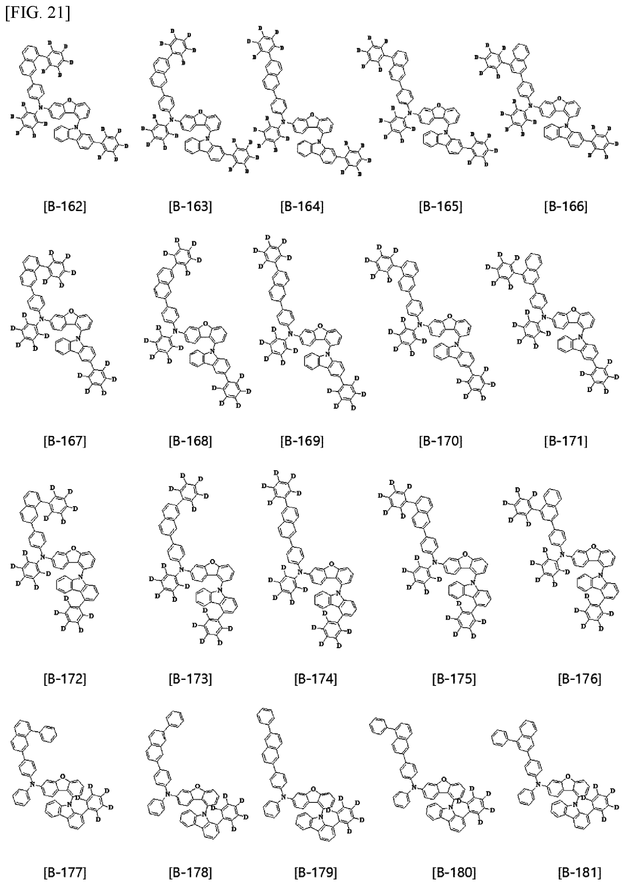

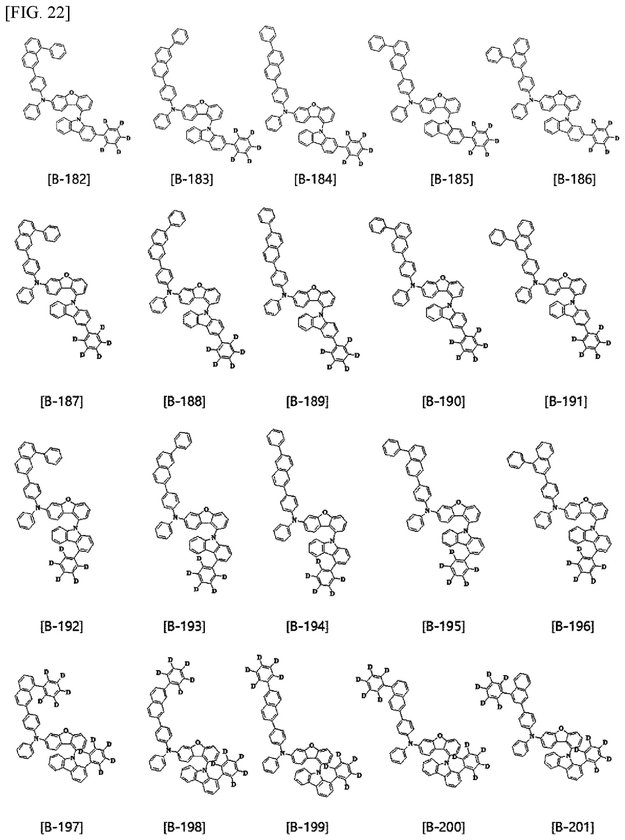

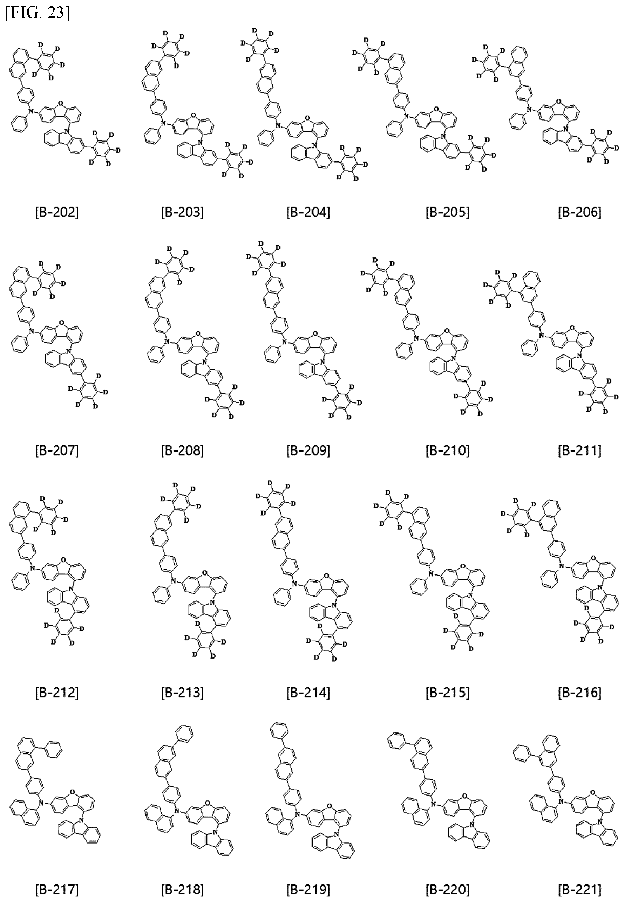

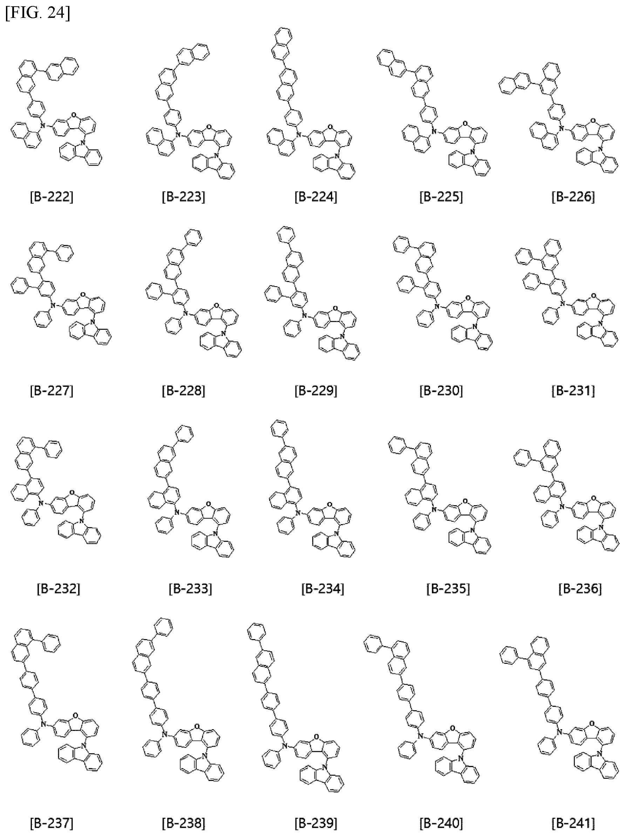

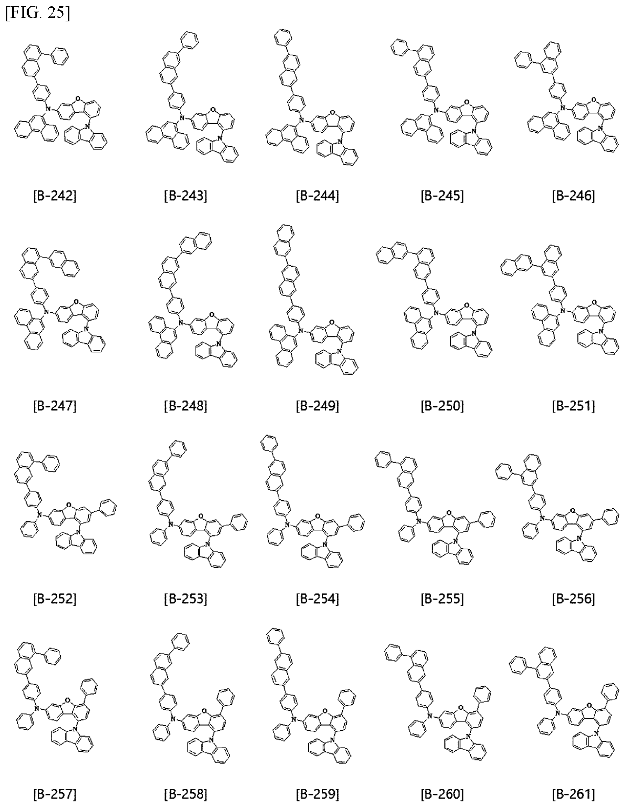

Among the arylamine compounds represented by the general formula (1), which are preferably used in the organic EL element of the present disclosure, specific examples of preferable compounds are shown in FIGS. 1 to 26, but they are not limited to these compounds.

Purification of the arylamine compound represented by the general formula (1) can be carried out by known methods such as a purification method by column chromatography, an adsorption purification method by silica gel, activated carbon, activated clay, etc., a recrystallization or crystallization method by a solvent, a sublimation purification method, etc. It is desirable to finally perform purification using a sublimation purification method.

The compounds used in the organic EL element of the present disclosure were purified by column chromatography, adsorption purification by silica gel, activated carbon, activated clay, etc., recrystallization or crystallization by a solvent, or the like, and finally, by sublimation purification.

Identification of compounds can be performed by NMR analysis. As physical property values, melting point, glass transition point (Tg), and work function are preferably measured. The melting point is an indicator of deposition properties, the glass transition point (Tg) is an indicator of the stability of the thin film state, and the work function is an indicator of hole injection properties, hole transport properties, or electron blocking properties.

The melting point and the glass transition point (Tg) can be measured, for example, with a high-sensitivity differential scanning calorimeter (DSC3100SA, manufactured by Bruker AXS) using powder.

The work function can be determined, for example, by manufacturing a 100 nm thin film on an ITO substrate and using an ionization potential measurement device (PYS-202, manufactured by Sumitomo Heavy Industries, Ltd.).

The structure of the organic EL element of the present disclosure may include a structure in which an anode, a hole injection layer, a hole transport layer, a light emitting layer, an electron transport layer, an electron injection layer and a cathode are sequentially formed on a substrate, a structure in which an electron blocking layer is formed between the hole transport layer and the light emitting layer, and a structure in which a hole blocking layer is formed between the light emitting layer and the electron transport layer. In these multilayer structures, it is possible for one organic layer to play the role of several layers, and for example, one organic layer can have a structure that also serves as a hole injection layer and a hole transport layer, or it can have a structure that also serves as an electron injection layer and an electron transport layer. In addition, it is possible to have a configuration in which two or more organic layers with the same function are stacked, and a configuration in which two layers of a hole transport layer are stacked, a configuration in which two layers of a light emitting layer are stacked, and a configuration in which two layers of an electron transport layer are stacked can be used.

As the anode of the organic EL element of the present disclosure, an electrode material with a large work function such as ITO or gold is used.

As a material for the hole injection layer of the organic EL element of the present disclosure, it is preferable to use an arylamine compound having only one triphenylamine structure in the molecule, in addition to the arylamine compound represented by the general formula (1) of the present disclosure. In addition, a porphyrin compound represented by copper phthalocyanine, a starburst type triphenylamine derivative, an arylamine compound having two or more triphenylamine structures or carbazolyl structures in the molecule, each of which has a structure linked by a divalent group that does not contain a single bond or heteroatom, acceptor heterocyclic compounds such as hexacyanoazatriphenylene, and coating-type polymer materials can be used. These materials can be formed into thin films by known methods such as vapor deposition, spin coating, and inkjet methods.

As a material for the hole injection layer and hole transport layer of the organic EL element of the present disclosure, it is preferable to use an arylamine compound having only one triphenylamine structure in the molecule, in addition to the arylamine compound represented by the general formula (1) of the present disclosure. In addition, benzidine derivatives such as N,N'-diphenyl-N,N'-di(m-tolyl)-benzidine (TPD), N,N'-diphenyl-N,N'-di(α-naphthyl)-benzidine (NPD), and N,N,N',N'-tetrabiphenylylbenzidine, 1,1-bis[(di-4-tolylamino)phenyl]cyclohexane (TAPC), and an arylamine compound having two or more triphenylamine structures or carbazolyl structures in the molecule, each of which has a structure linked by a divalent group that does not contain a single bond or heteroatom can be used. These materials may be formed into a film individually, but may be formed into a film by mixing multiple types, or each of them may be used as a single layer. In addition, it may be a laminated structure of layers formed by forming a film of these materials alone, a laminated structure of layers formed by mixing these materials, or a laminated structure of a layer formed of these materials alone and a layer formed by mixing a plurality of types. In addition, as a material for the hole injection/transport layer, a coating type polymer material such as poly(3,4-ethylenedioxythiophene) (PEDOT)/poly(styrenesulfonate) (PSS) can be used. These materials may be formed into thin films by known methods such as vapor deposition, spin coating, and inkjet methods.

In addition, in the hole injection layer or the hole transport layer, a material commonly used in these layers is preferably P-doped with trisbromophenylamine hexachloroantimony, radialene derivatives (see, e.g., Patent Document 6), etc. In addition, polymer compounds having the structure of benzidine derivatives such as TPD in their partial structures, etc. can be used.

As a material for the electron blocking layer of the organic EL element of the present disclosure, it is preferable to use the arylamine compound represented by the general formula (1) of the present disclosure. In addition, compounds having an electron blocking function, such as compounds having a triphenylsilyl group and a triarylamine structure represented by carbazole derivatives such as 4,4',4"-tri(N-carbazolyl)triphenylamine (TCTA), 9,9-bis[4-(carbazol-9-yl)phenyl] fluorene, 1,3-bis(carbazol-9-yl)benzene (mCP), and 2,2-bis(4-carbazol-9-ylphenyl)adamantane (Ad-Cz), and 9-[4-(carbazole-9-yl)phenyl]-9-[4-(triphenylsilyl)phenyl]-9H-fluorene, can be used. These materials may also serve as materials for the hole transport layer. These materials may be formed into a film individually, but may be formed into a film by mixing multiple types, or each of them may be used as a single layer. In addition, it may be a laminated structure of layers formed by forming a film of these materials alone, a laminated structure of layers formed by mixing these materials, or a laminated structure of a layer formed of these materials alone and a layer formed by mixing a plurality of types. These materials may be formed into thin films by known methods such as vapor deposition, spin coating, and inkjet methods.

As a material for the light emitting layer of the organic EL element of the present disclosure, it is preferable to use the arylamine compound represented by the general formula (1) of the present disclosure. In addition, metal complexes of quinolinol derivatives including tris(8-quinolinolato)aluminum (Alq₃), and various metal complexes, anthracene derivatives, bis-styrylbenzene derivatives, pyrene derivatives, oxazole derivatives, polyparaphenylenevinylene derivatives, etc. can be used. In addition, the light emitting layer may be composed of a host material and a dopant material, and the arylamine compound represented by the general formula (1) of the present disclosure or an anthracene derivative is preferably used as the host material. In addition to the materials above, heterocyclic compounds having an indole ring as a partial structure of a condensed ring, heterocyclic compounds having a carbazole ring as a partial structure of a condensed ring, carbazole derivatives, thiazole derivatives, benzimidazole derivatives, polydialkylfluorene derivatives, etc. may be used. Also, heterocyclic compounds having S, B, N, etc. as ring constituent elements are preferably used as the dopant material. In addition, quinacridone, coumarin, rubrene, perylene and their derivatives, benzopyran derivatives, rhodamine derivatives, aminostyryl derivatives, etc. may be used. These materials may be formed into a film individually, but may be formed into a film by mixing multiple types, or each of them may be used as a single layer. In addition, it may be a laminated structure of layers formed by forming a film of these materials alone, a laminated structure of layers formed by mixing these materials, or a laminated structure of a layer formed of these materials alone and a layer formed by mixing a plurality of types. These materials may be formed into thin films by known methods such as vapor deposition, spin coating, and inkjet methods.

In addition, it is also possible to use a phosphorescent emitter as a light emitting material. A phosphorescent emitter of a metal complex such as iridium or platinum may be used as the phosphorescent emitter. For example, the phosphorescent emitter may include a green phosphorescent emitter such as Ir(ppy)₃, a blue phosphorescent emitter such as FIrpic or FIr₆, a red phosphorescent emitter such as Btp₂Ir(acac), etc. The host material at this time may include, as a host material with hole injection/transport properties, the arylamine compound of the present disclosure in addition to carbazole derivatives such as 4,4'-di(N-carbazolyl)biphenyl (CBP), TCTA, and mCP, and may include, as a host material with electron transport properties, p-bis(triphenylsilyl)benzene (UGH2), 2,2',2"-(1,3,5-phenylene)-tris(1-phenyl-1H-benzimidazole) (TPBI), or the like. A high-performance organic EL element may be manufactured by using such materials.

In order to avoid concentration quenching, doping of the host material of the phosphorescent light emitting material is preferably carried out by co-deposition in the range of 1 to 30 weight percent with respect to the entire light emitting layer.

In addition, it is also possible to use a material that emits delayed fluorescence, such as CDCB derivatives such as PIC-TRZ, CC2TA, PXZ-TRZ, and 4CzIPN as the light emitting material (see, e.g., Non-Patent Document 3). These materials may be formed into thin films by known methods such as vapor deposition, spin coating, and inkjet methods.

As a material for the hole blocking layer of the organic EL element of the present disclosure, phenanthroline derivatives such as basocuproine (BCP), metal complexes of quinolinol derivatives such as bis(2-methyl-8-quinolinolate)-4-(phenylphenolato)aluminum (BAlq), various rare earth complexes, and compounds having a hole blocking effect, such as oxazole derivatives, triazole derivatives, and triazine derivatives, may be used. These materials may also serve as materials for the electron transport layer. These materials may be formed into a film individually, but may be formed into a film by mixing multiple types, or each of them may be used as a single layer. In addition, it may be a laminated structure of layers formed by forming a film of these materials alone, a laminated structure of layers formed by mixing these materials, or a laminated structure of a layer formed of these materials alone and a layer formed by mixing a plurality of types. These materials may be formed into thin films by known methods such as vapor deposition, spin coating, and inkjet methods.

As a material for the electron transport layer of the organic EL element of the present disclosure, it is preferable to use benzimidazole derivatives, anthracene derivatives, pyrimidine derivatives, or triazine derivatives. In addition, metal complexes of quinolinol derivatives including Alq₃ and BAlq, various metal complexes, triazole derivatives, oxadiazole derivatives, pyridine derivatives, thiadiazole derivatives, carbodiimide derivatives, quinoxaline derivatives, pyridoindole derivatives, phenanthroline derivatives, silole derivatives, etc. may be used. These materials may be formed into a film individually, but may be formed into a film by mixing multiple types, or each of them may be used as a single layer. In addition, it may be a laminated structure of layers formed by forming a film of these materials alone, a laminated structure of layers formed by mixing these materials, or a laminated structure of a layer formed of these materials alone and a layer formed by mixing a plurality of types. These materials may be formed into thin films by known methods such as vapor deposition, spin coating, and inkjet methods.

As a material for the electron injection layer of the organic EL element of the present disclosure, alkali metal salts such as lithium fluoride and cesium fluoride, alkaline earth metal salts such as magnesium fluoride, metal complexes of quinolinol derivatives such as lithium quinolinol, metal oxides such as aluminum oxide, and metals such as ytterbium (Yb), samarium (Sm), calcium (Ca), strontium (Sr), and cesium (Cs) may be used. The electron injection layer may be omitted depending on the desirable choice of the electron transport layer and cathode.

In addition, in the electron injection layer and the electron transport layer, a material commonly used in these layers may be N-doped with metals such as cesium.

In the cathode of the organic EL element of the present disclosure, a metal with a low work function, such as aluminum, and an alloy with a lower work function, such as a magnesium silver alloy, a magnesium indium alloy, and an aluminum magnesium alloy, are used as electrode materials.

### [MODE FOR CARRYING OUT THE INVENTION]

### EXAMPLES

Hereinafter, embodiments of the present disclosure will be specifically described through Examples, but the present disclosure is not limited to the following Examples as long as the gist is not exceeded. The reagents described in the synthesis examples were manufactured by Tokyo Chemical Industry Co., Ltd., Sigma-Aldrich, Alfa Aesar, etc. In addition, all reactions in the synthesis examples were performed using reaction vessels equipped with a cooling pipe, a stirring device, and a thermometer. Further, the compounds in the synthesis examples were identified by ¹H-NMR analysis (Bruker's nuclear magnetic resonance spectrometer, type: Ascend^{™} 400 MHz).

### [Example 1]

### <Synthesis of N-phenyl-N-{4-(1-phenyl-naphthalen-7-yl)phenyl}-{1-(9H-carbazol-9-yl)dibenzo[b,d]furan-8-yl}-amine (Compound: A-1)>

To a nitrogen-purged reaction vessel,
9.0 g of N-phenyl-N-{4-(1-phenyl-naphthalen-7-yl)phenyl}-amine, 9.8 g of 9-(8-chlorodibenzo[b,d]furan-1-yl)-9H-carbazole, 0.4 g of tris(dibenzylideneacetone)palladium(0), 0.2 g of tri-tert-butylphosphine, and 2.8 g of sodium tert-butoxy were added, and the mixture was refluxed and stirred overnight under a xylene solvent.

After checking the completion of the reaction, the filtrate obtained by filtration was concentrated to obtain a crude product. The obtained crude product was subjected to recrystallization and purification using a toluene solvent to obtain 6.5 g of a white powder of N-phenyl-N-{4-(1-phenyl-naphthalen-7-yl)phenyl}-{1-(9H-carbazol-9-yl)dibenzo[b,d]furan-8-yl}-amine (Compound: A-1) (yield: 38.2%).

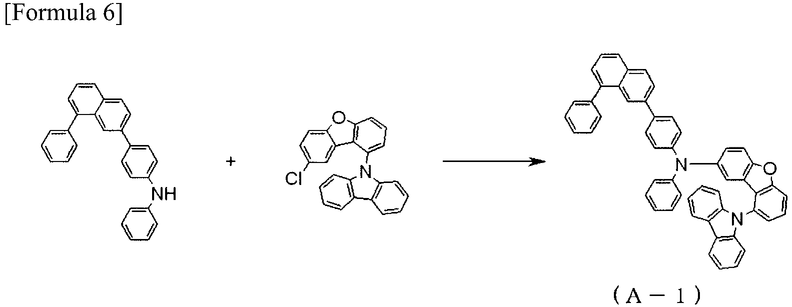

The structure of the obtained white powder was identified using NMR.

The following 34 hydrogen signals were detected by ¹H-NMR (DMSO-d₆).
δ (ppm) =8. 13 (1H), 8. 04 (1H), 8. 01(1H), 7. 96(3H),
7. 84 (1H), 7. 8 2 (1H), 7. 70 (1H), 7. 65-7. 5 5 (6H), 7.
4 9 (2H), 7. 3 3 (2H), 7. 24 (2H), 7. 14 (3H), 7. 04 (4H),
6. 95 (1H), 6. 68 (2H), 6. 61 ( 2H), 5. 91 (1H).

### [Example 2]

### <Synthesis of N-phenyl-N-{4-(1-phenyl-naphthalen-3-yl)phenyl}-{1-(9H-carbazol-9-yl)dibenzo[b,d]furan-8-yl}-amine (Compound: A-5)>

To a nitrogen-purged reaction vessel,
9.0 g of N-phenyl-N-{4-(1-phenyl-naphthalen-3-yl)phenyl}-amine, 9.4 g of 9-(8-chlorodibenzo[b,d]furan-1-yl)-9H-carbazole, 0.4 g of tris(dibenzylideneacetone)palladium(0), 0.2 g of tri-tert-butylphosphine, and 2.8 g of sodium tert-butoxy were added, and the mixture was refluxed and stirred overnight under a xylene solvent.

After checking the completion of the reaction, the filtrate obtained by filtration was concentrated to obtain a crude product. The obtained crude product was subjected to crystallization and purification using a toluene/acetone mixed solvent to obtain 13.8 g of a white powder of N-phenyl-N-{4-(1-phenyl-naphthalen-3-yl)phenyl}-{1-(9H-carbazol-9-yl)dibenzo[b,d]furan-8-yl}-amine (Compound: A-5) (yield: 81.0%).

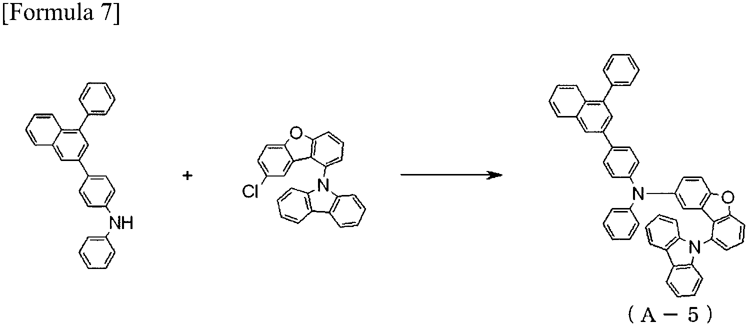

The structure of the obtained white powder was identified using NMR.

The following 34 hydrogen signals were detected by ¹H-NMR (DMSO-d₆).
δ (ppm) =8. 21 (1H), 8. 10 (1H), 8. 06 (2H), 7. 97 (1H),
7. 82 (2H), 7. 7 3 (1H), 7. 7 1 (1H), 7. 64-7. 55 (8H), 7.
51 (2H), 7. 3 1 (2H), 7. 19 (1H), 7. 15 (4H), 7. 04 (2H),
6. 98 (1H), 6. 69 (4H), 5. 98 (1H).

### [Example 3]

### <Synthesis of N-([1,1']biphenyl-4-yl)-N-{4-(1-phenyl-naphthalen-7-yl)phenyl}-{1-(9H-carbazol-9-yl)dibenzo[b,d]furan-8-yl}-amine (Compound: A-36)>

To a nitrogen-purged reaction vessel,
10.0 g of N-([1,1']biphenyl-4-yl)-N-{4-(1-phenyl-naphthalen-7-yl)phenyl}-amine, 8.6 g of 9-(8-chlorodibenzo[b,d]furan-1-yl)-9H-carbazole, 0.4 g of tris(dibenzylideneacetone)palladium(0), 0.2 g of tri-tert-butylphosphine, and 2.6 g of sodium tert-butoxy were added, and the mixture was refluxed and stirred overnight under a xylene solvent.

After checking the completion of the reaction, the filtrate obtained by filtration was concentrated to obtain a crude product. The obtained crude product was subjected to recrystallization and purification using a toluene solvent to obtain 14.9 g of a white powder of N-([1,1']biphenyl-4-yl)-N-{4-(1-phenyl-naphthalen-7-yl)phenyl}-{1-(9H-carbazol-9-yl)dibenzo[b,d]furan-8-yl}-amine (Compound: A-36) (yield: 85.6%).

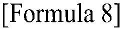

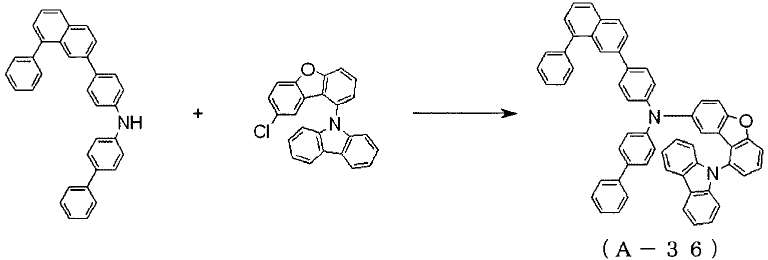

The structure of the obtained white powder was identified using NMR.

The following 38 hydrogen signals were detected by ¹H-NMR (DMSO-d₆).
δ (ppm) =8. 10 (1H), 8. 03 (1H), 7. 9 9 (1H), 7. 96 (1H),
7. 92 (2H), 7. 8 2 (1H), 7. 8 1 (1H), 7. 7 0 (1H), 7. 6 4 - 7.
52 (8H), 7. 46 (4H), 7. 40 (2H), 7. 33 (3H), 7. 21 (3H),
7. 29-6. 95 (4H), 6. 69 (2H), 6. 66 (2H), 5. 93 (1H),

### [Example 4]

### <Synthesis of N-phenyl-N-{4-(1-phenyl-naphthalen-7-yl)phenyl}-{1-(9H-carbazol-9-yl)dibenzo[b,d]furan-7-yl}-amine (Compound: B-1)>

To a nitrogen-purged reaction vessel,
4.0 g of N-phenyl-N-{4-(1-phenyl-naphthalen-7-yl)phenyl}-amine, 4.0 g of 9-(7-chlorodibenzo[b,d]furan-1-yl)-9H-carbazole, 0.2 g of tris(dibenzylideneacetone)palladium(0), 0.1 g of tri-tert-butylphosphine, and 2.1 g of sodium tert-butoxy were added, and the mixture was refluxed and stirred overnight under a xylene solvent.

After checking the completion of the reaction, methanol was added to the system to precipitate solid, which was then filtered to obtain a crude product. The obtained crude product was subjected to crystallization and purification using a tetrahydrofuran/ethyl acetate mixed solvent to obtain 4.3 g of a white powder of N-phenyl-N-{4-(1-phenyl-naphthalen-7-yl)phenyl}-{1-(9H-carbazol-9-yl)dibenzo[b,d]furan-7-yl}-amine (Compound: B-1) (yield: 56.3%).

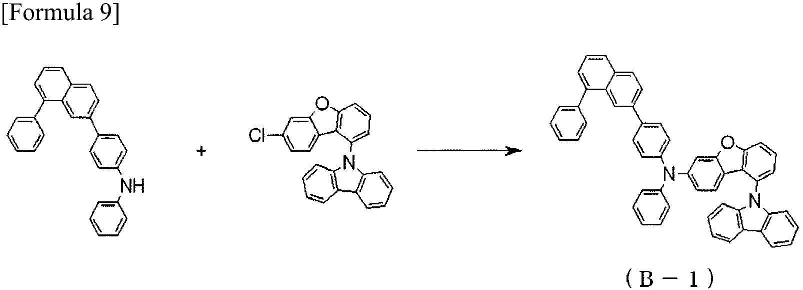

The structure of the obtained white powder was identified using NMR.

The following 34 hydrogen signals were detected by ¹H-NMR (DMSO-d₆).
δ (ppm) =8. 32 (2H), 8. 06 (1H), 7. 97 (III), 7. 95 (1H),
7.89 (1H), 7. 79 (1H), 7. 74 (1H), 7. 57 (2H), 7.54-7.
42 (8H), 7. 37 (2H), 7. 29 (4H), 7. 22 (1H), 7. 08 (3H),
7. 01 (4H), 6. 53 (1H), 6. 00 (1H),

### [Example 5]

### <Synthesis of N-([1,1']biphenyl-4-yl)-N-{4-(1-phenyl-naphthalen-3-yl)phenyl}-{1-(9H-carbazol-9-yl)dibenzo[b,d]furan-7-yl}-amine (Compound: B-40)>

To a nitrogen-purged reaction vessel,
10.0 g of N-([1,1']biphenyl-4-yl)-N-{4-(1-phenyl-naphthalen-3-yl)phenyl}-amine, 8.2 g of 9-(7-chlorodibenzo[b,d]furan-1-yl)-9H-carbazole, 0.4 g of tris(dibenzylideneacetone)palladium(0), 0.2 g of tri-tert-butylphosphine, and 4.3 g of sodium tert-butoxy were added, and the mixture was refluxed and stirred overnight under a xylene solvent.

After checking the completion of the reaction, methanol was added to the system to precipitate solid, which was then filtered to obtain a crude product. The obtained crude product was isolated by column chromatography (carrier: silica gel, eluent: dichloromethane/n-heptane) and subjected to crystallization and purification using an acetone/n-heptane mixed solvent to obtain 5.0 g of a white powder of N-([1,1']biphenyl-4-yl)-N-{4-(1-phenyl-naphthalen-3-yl)phenyl}-{1-(9H-carbazol-9-yl)dibenzo[b,d]furan-7-yl}-amine (Compound: B-40) (yield: 28.7%).

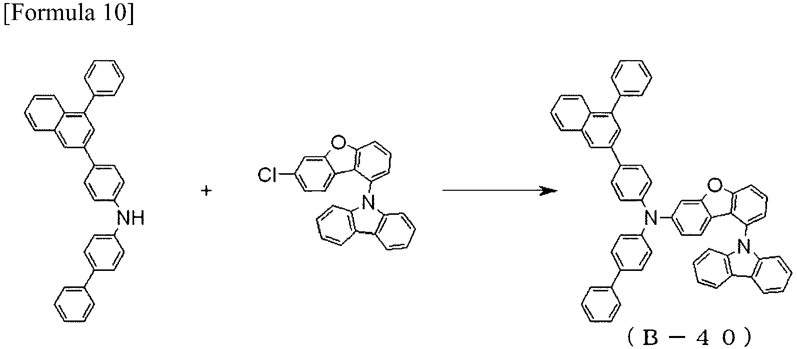

The structure of the obtained white powder was identified using NMR.

The following 38 hydrogen signals were detected by ¹H-NMR (DMSO-d₆).
δ (ppm) =8. 21 (2H), 8. 17 (1H), 8. 02 (1H), 7. 8 9 (1H),
7.78 (1H), 7. 7 3 (1H), 7. 7 1 (2H), 7. 6 3 (1H), 7. 59-7.
43 (12H), 7. 40 ( 2H), 7. 36-7. 2 4 (6H), 7. 04 (6H), 6.
51 (1H), 5. 81 (1H),

### [Example 6]

### <Synthesis of N-phenyl-N-{4-(1-phenyl-naphthalen-7-yl)phenyl}-{1-(2-phenyl-9H-carbazol-9-yl)dibenzo[b,d]furan-7-yl}-amine (Compound: B-86)>

To a nitrogen-purged reaction vessel,
10.0 g of N-phenyl-N-{4-(1-phenyl-naphthalen-7-yl)phenyl}-amine, 13.1 g of 2-phenyl-9-(7-chlorodibenzo[b,d]furan-1-yl)-9H-carbazole, 0.5 g of tris(dibenzylideneacetone)palladium(0), 0.2 g of tri-tert-butylphosphine, and 3.1 g of sodium tert-butoxy were added, and the mixture was refluxed and stirred overnight under a xylene solvent.

After checking the completion of the reaction, the filtrate obtained by filtration was concentrated to obtain a crude product. The obtained crude product was subjected to crystallization and purification using an ethyl acetate/acetone mixed solvent to obtain 14.8 g of a white powder of N-phenyl-N-{4-(1-phenyl-naphthalen-7-yl)phenyl}-{1-(2-phenyl-9H-carbazol-9-yl)dibenzo[b,d]furan-7-yl}-amine (Compound: B-86) (yield: 70.6%).

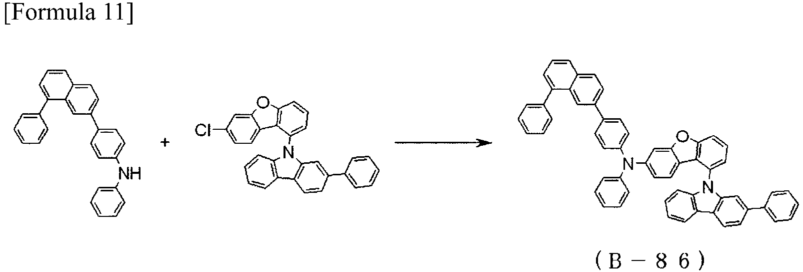

The structure of the obtained white powder was identified using NMR.

The following 38 hydrogen signals were detected by ¹H-NMR (DMSO-d₆).
δ (ppm) =8. 41 (1H), 8.36 (1H), 8. 07 (1H), 7. 98 (1H),
7. 9 4 (1H), 7. 90 (1H), 7. 89 (1H), 7. 7 6 (1H), 7. 66 (1
H), 7. 6 2 (1H), 7. 5 8 (1H), 7. 5 6 - 7. 4 3 (10H), 7. 40 (3
H), 7. 36-7. 2 4 (6H), 7.12 (1H), 7. 08 (1H), 7. 02 (4H),
6. 59 (1H), 6. 1 1 (1H).

### [Example 7]

### <Synthesis of 9-(9H-carbazol-9-yl)-N-{4-(7,8-diphenylnaphthalen-2-yl)phenyl}-N-phenyldibenzo[b,d]furan-2-amine (Compound: A-236)>

To a nitrogen-purged reaction vessel,
7.0 g of 9-(9H-carbazol-9-yl)-N-phenyldibenzo[b,d]furan-2-amine, 6.8 g of 7-(4-chlorophenyl)-1,2-diphenylnaphthalene, 0.3 g of tris(dibenzylideneacetone)palladium(0), 0.1 g of tri-tert-butylphosphine, and 1.9 g of sodium tert-butoxy were added, and the mixture was refluxed and stirred overnight under a xylene solvent.

After checking the completion of the reaction, toluene was added to dissolve the precipitated solid, and then the reaction mixture was filtered. The crude product obtained by concentrating the filtrate was subjected to crystallization and purification using a toluene/acetone mixed solvent to obtain 8.3 g of a white powder of 9-(9H-carbazol-9-yl)-N-{4-(7,8-diphenylnaphthalen-2-yl)phenyl}-N-phenyldibenzo[b,d]furan-2-amine (Compound: A-236) (yield: 64.6%).

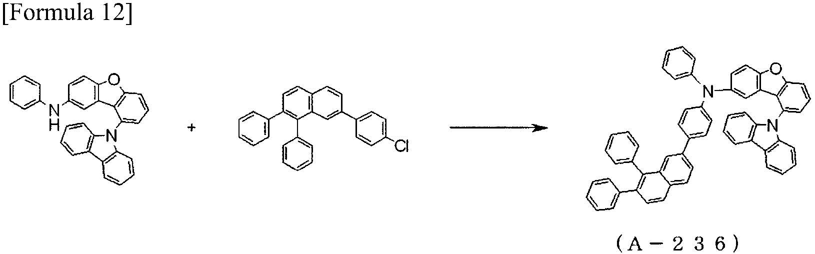

The structure of the obtained white powder was identified using NMR.

The following 38 hydrogen signals were detected by ¹H-NMR (CDCl₃).
δ (ppm) =8. 04 (1H), 7. 99 (1H), 7. 94 (1H), 7. 90 (2H),
7. 7 8 (1H), 7. 7 4 (1H), 7. 6 7 (1H), 7. 6 1 (1H), 7. 49 (2
H), 7. 3 7 - 7. 18 (15H), 7. 12-7. 05 (6H), 6. 9 2 (1H), 6.
77 (2H), 6. 70 (2H), 6. 11 (1H),

### [Example 8]

### <Synthesis of N-{4-(1,2'-binaphthalen-7-yl)phenyl}-9-(9H-carbazol-9-yl)-N-phenyldibenzo[b,d]furan-3-amine (Compound: B-6)>

To a nitrogen-purged reaction vessel,
6.2 g of 4-(1,2'-binaphthalen-7-yl)-N-phenylaniline, 5.4 g of 9-(7-chlorodibenzo[b,d]furan-1-yl)-9H-carbazole, 0.3 g of tris(dibenzylideneacetone)palladium(0), 0.1 g of tri-tert-butylphosphine, and 2.8 g of sodium tert-butoxy were added, and the mixture was refluxed and stirred overnight under a xylene solvent.

After checking the completion of the reaction, methanol was added, and the precipitated solid was filtered. The crude product obtained was subjected to crystallization and purification using a tetrahydrofuran/methanol mixed solvent to obtain 7.5 g of a white powder of N-{4-(1,2'-binaphthalen-7-yl)phenyl}-9-(9H-carbazol-9-yl)-N-phenyldibenzo[b,d]furan-3-amine (Compound: B-6) (yield: 67.7%).

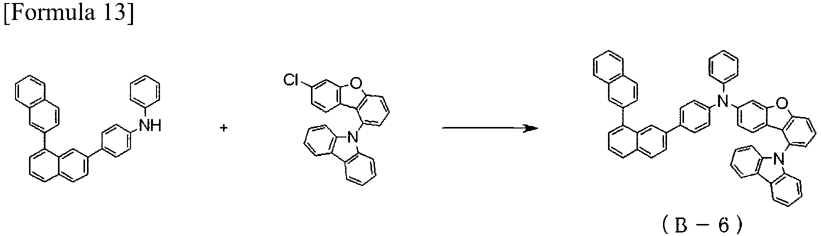

The structure of the obtained white powder was identified using NMR.

The following 36 hydrogen signals were detected by ¹H-NMR (CDCl₃).
δ (ppm) =8. 22 (2H), 8. 10 (1H), 8. 01-7. 91 (6H), 7.
7 5 (1H), 7. 7 0 (1H), 7. 6 7 (1H), 7. 61-7. 5 5 (5H), 7. 4
8 (1H), 7. 4 4 (2H), 7.38 (2H), 7.32 (2H), 7. 25-7.2 1
(3H), 7. 17 (2H), 7. 09-7. 02 (5H), 6. 6 3 (1H), 6. 20 (1
   H).

### [Example 9]

### <Synthesis of N-{4-([1,1'-binaphthalen]-7-yl)phenyl}-9-(9H-carbazol-9-yl)-N-phenyldibenzo[b,d]furan-3-amine (Compound: B-11)>

To a nitrogen-purged reaction vessel,
10.0 g of 4-([1,1'-binaphthalen]-7-yl)-N-phenylaniline, 8.7 g of 9-(7-chlorodibenzo[b,d]furan-1-yl)-9H-carbazole, 0.4 g of tris(dibenzylideneacetone)palladium(0), 0.2 g of tri-tert-butylphosphine, and 2.7 g of sodium tert-butoxy were added, and the mixture was refluxed and stirred overnight under a xylene solvent.

After checking the completion of the reaction, toluene was added to dissolve the precipitated solid, and then the reaction mixture was filtered. The crude product obtained by concentrating the filtrate was subjected to crystallization and purification using a toluene/acetone mixed solvent to obtain 12.7 g of a white powder of N-{4-([1,1'-binaphthalen]-7-yl)phenyl}-9-(9H-carbazol-9-yl)-N-phenyldibenzo[b,d]furan-3-amine (Compound: B-11) (yield: 71.1%).

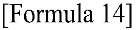

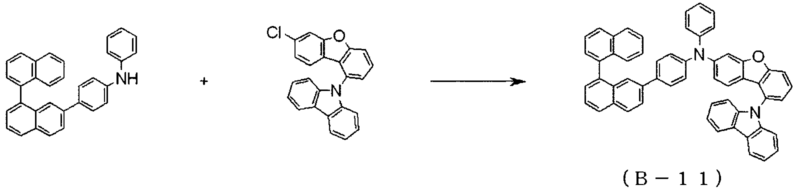

The structure of the obtained white powder was identified using NMR.

The following 36 hydrogen signals were detected by ¹H-NMR (CDCl₃).
δ (ppm) =8. 22 (2H), 8. 02-7. 95 (4H), 7. 7 1 (1H), 7.
67 (1H), 7. 63-7. 47 (9H), 7. 39-7. 30 (5H), 7. 26-7.
16 (7H), 7. 04 (3H), 6. 98 (2H), 6. 59(1H), 6. 19(1H).

### [Example 10]

### <Synthesis of N-{4-(8-([1,1'-biphenyl]-2-yl)naphthalen-2-yl)phenyl}-9-(9H-carbazol-9-yl)-N-phenyldibenzo[b,d]furan-3-amine (Compound: B-31)>

To a nitrogen-purged reaction vessel,
6.5 g of 4-(8-([1,1'-biphenyl]-2-yl)naphthalen-2-yl)-N-phenylaniline, 5.6 g of 9-(7-chlorodibenzo[b,d]furan-1-yl)-9H-carbazole, 0.1 g of tris(dibenzylideneacetone)palladium(0), 0.1 g of tri-tert-butylphosphine, and 2.1 g of sodium tert-butoxy were added, and the mixture was heated to 98°C, and refluxed and stirred overnight under a xylene solvent.

After checking the completion of the reaction, toluene was added to dissolve the precipitated solid, and then the reaction mixture was filtered. The obtained crude product was isolated by column chromatography (carrier: silica gel, eluent: dichloromethane/n-heptane) to obtain 8.5 g of a white powder of N-{4-(8-([1,1'-biphenyl]-2-yl)naphthalen-2-yl)phenyl}-9-(9H-carbazol-9-yl)-N-phenyldibenzo[b,d]furan-3-amine (Compound: B-31) (yield: 75.2%).

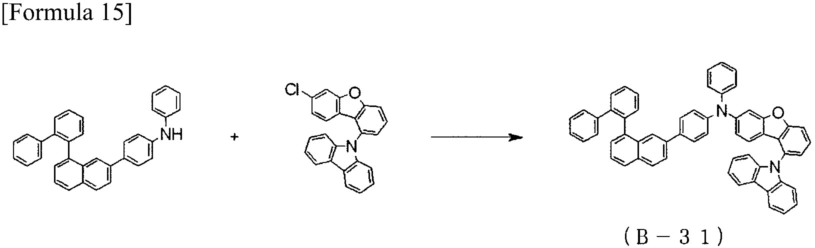

The structure of the obtained white powder was identified using NMR.

The following 38 hydrogen signals were detected by ¹H-NMR (CDCl₃).
δ (ppm) =8. 23 (2H), 7. 87 (1H), 7. 79 (1H), 7. 77 (1H),
7. 6 9 (1H), 7. 64-7. 4 7 (7H), 7. 4 3-7. 31 (7H), 7. 2 8
-7.24 (4H), 7. 19 ( 2H), 7. 14-7. 0 3 (10H), 6. 6 6 (1H),
6. 23 (1H).

### [Example 11]

### <Synthesis of 9-(9H-carbazol-9-yl)-N-(phenyl-d)- N- {4-(8-(phenyl-d)naphthalen-2-yl)phenyl}dibenzo[b,d]furan-3-amine (Compound: B-146)>

To a nitrogen-purged reaction vessel,
8.0 g of N-{4-(8-(phenyl-d)naphthalen-2-yl)phenyl}(benzene-d)amine, 7.7 g of 9-(7-chlorodibenzo[b,d]furan-1-yl)-9H-carbazole, 0.4 g of tris(dibenzylideneacetone)palladium(0), 0.2 g of tri-tert-butylphosphine, and 4.0 g of sodium tert-butoxy were added, and the mixture was refluxed and stirred overnight under a xylene solvent.

After checking the completion of the reaction, methanol was added, and the precipitated solid was filtered. The obtained crude product was subjected to crystallization and purification using a tetrahydrofuran/ethyl acetate mixed solvent to obtain 6.5 g of a white powder of 9-(9H-carbazol-9-yl)-N-(phenyl-d)-N- {4-(8-(phenyl-d)naphthalen-2-yl)phenyl}dibenzo[b,d]furan-3-amine (Compound: B-146) (yield: 43.6%).

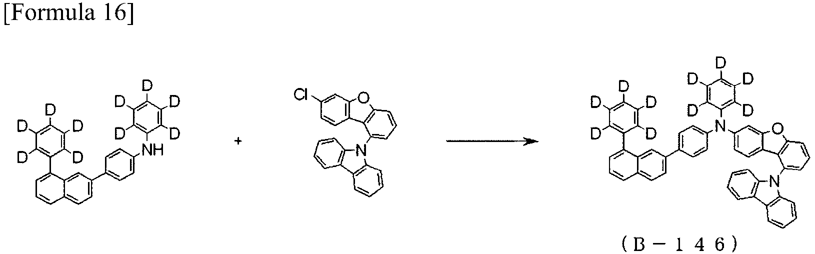

The structure of the obtained white powder was identified using NMR.

The following 24 hydrogen signals were detected by ¹H-NMR (CDCl₃).
δ (ppm) -8. 23 (2H), 8.08 (1H), 7. 98 (111), 7. 89 (III),
7. 7 4 (1H), 7. 6 9 (1H), 7. 6 0 (1H), 7. 5 4 (1H), 7. 5 0 - 7.
45 (4H), 7. 3 9 (2H), 7. 33 (2H), 7. 27 (1H), 7. 19 (2H),
7. 1 1 (2H), 6. 6 5 (1H), 6. 2 2 (1H)_{∘}

### [Example 12]

### <Synthesis of 9-(9H-carbazol-9-yl)-N-phenyl-N-(4'-(8-phenylnaphthalen-2-yl)-[1,1'-biphenyl]-4-yl)dibenzo[b,d]furan-3-amine (Compound: B-237)>

### To a nitrogen-purged reaction vessel,

10.0 g of N-phenyl-4'-(8-phenylnaphthalen-2-yl)-[1,1'-biphenyl]-4-amine, 8.2 g of 9-(7-chlorodibenzo[b,d]furan-1-yl)-9H-carbazole, 0.4 g of tris(dibenzylideneacetone)palladium(0), 0.4 g of Sphos, and 4.3 g of sodium tert-butoxy were added, and the mixture was refluxed and stirred overnight under a xylene solvent.

After checking the completion of the reaction, methanol was added, and the precipitated solid was filtered. The obtained crude product was subjected to crystallization and purification using a tetrahydrofuran/ethyl acetate mixed solvent to obtain 4.1 g of a white powder of 9-(9H-carbazol-9-yl)-N-phenyl-N-(4'-(8-phenylnaphthalen-2-yl)-[1,1'-biphenyl]-4-yl)dibenzo[b,d]furan-3-amine (Compound: B-237) (yield: 23.6%).

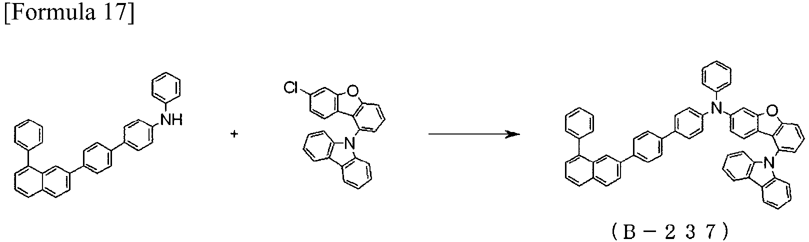

The structure of the obtained white powder was identified using NMR.

The following 38 hydrogen signals were detected by ¹H-NMR (CDCl₃).
δ (ppm) = 8. 24 (2H), 8. 19 (1H), 8. 03 (1H), 7. 9 2 (1H),
7. 83 (1H), 7. 6 9 (3H), 7. 63-7. 46 (13H), 7. 40 (2II),
7. 3 3 (2H), 7. 30-7. 26 (3H), 7. 20 (2H), 7. 14 (4H), 7.
08 (1H), 6. 67 (1H), 6. 24 (1H)_{∘}

### [Example 13]

### <Synthesis of 9-(9H-carbazol-9-yl)-N-phenyl-N-{4-(8-(phenyl-d)naphthalen-2-yl)phenyl-d}dibenzo[b,d]furan-3-amine (Compound: B-262)>

To a nitrogen-purged reaction vessel,
8.3 g of N-phenyl-4-(8-(phenyl-d)naphthalen-2-yl)benzene-damine, 8.0 g of 9-(7-chlorodibenzo[b,d]furan-1-yl)-9H-carbazole, 0.4 g of tris(dibenzylideneacetone)palladium(0), 0.2 g of tri-tert-butylphosphine, and 4.2 g of sodium tert-butoxy were added, and the mixture was refluxed and stirred overnight under a xylene solvent.

After checking the completion of the reaction, methanol was added, and the precipitated solid was filtered. The obtained solid was isolated by column chromatography (carrier: silica gel, eluent: dichloromethane/n-heptane). The obtained crude product was subjected to crystallization and purification using a tetrahydrofuran/ethyl acetate mixed solvent to obtain 7.2 g of a white powder of 9-(9H-carbazol-9-yl)-N-phenyl-N-{4-(8-(phenyl-d)naphthalen-2-yl)phenyl-d}dibenzo[b,d]furan-3-amine (Compound: B-262) (yield: 46.5%).

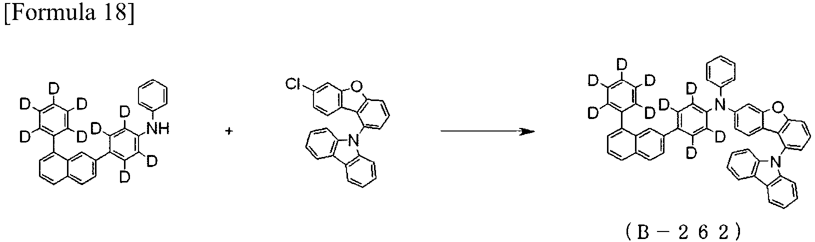

The structure of the obtained white powder was identified using NMR.

The following 25 hydrogen signals were detected by ¹H-NMR (CDCl₃).
δ (ppm) =8. 23 (2H), 8. 09 (1H), 7. 98 (1H), 7. 8 9 (1H),
7. 74 (1H), 7. 6 9 (1H), 7. 60 (1H), 7. 54 (1H), 7. 50-7.
45 (2H), 7. 39 (211), 7. 33 (2II), 7. 28-7. 24 (211), 7. 1
9(2H), 7. 12-7. 05 (4H), 6. 65 (1H), 6. 23 (1H),

### [Example 14]

### <Synthesis of 9-(9H-carbazol-9-yl)-N-phenyl-N-(8-phenylnaphthalen-2-yl)dibenzo[b,d]furan-3-amine (Compound: B-263)>

To a nitrogen-purged reaction vessel,
7.0 g of N,8-diphenylnaphthalen-2-amine, 9.2 g of 9-(7-chlorodibenzo[b,d]furan-1-yl)-9H-carbazole, 0.4 g of tris(dibenzylideneacetone)palladium(0), 0.8 g of tri-tert-butylphosphine, and 3.4 g of sodium tert-butoxy were added, and the mixture was heated to 98°C, and refluxed and stirred overnight under a xylene solvent.

After checking the completion of the reaction, toluene was added to dissolve the precipitated solid, and then the reaction mixture was filtered. The filtrate was concentrated, and the obtained solid was isolated by column chromatography (carrier: silica gel, eluent: dichloromethane/n-heptane). The obtained crude product was subjected to crystallization and purification using a tetrahydrofuran/acetone mixed solvent to obtain 7.0 g of a white powder of 9-(9H-carbazol-9-yl)-N-phenyl-N-(8-phenylnaphthalen-2-yl)dibenzo[b,d]furan-3-amine (Compound: B-263) (yield: 47.0%).

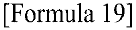

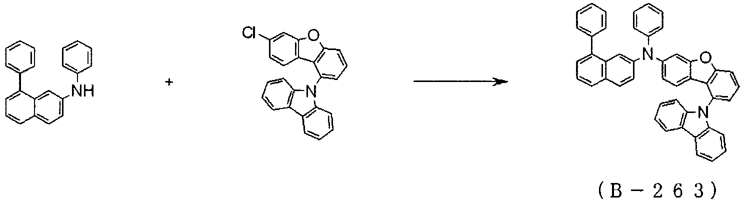

The structure of the obtained white powder was identified using NMR.

The following 30 hydrogen signals were detected by ¹H-NMR (CDCl₃).
δ (ppm) = 8. 23 (2H), 7. 7 7 (2H), 7. 6 9 (1H), 7. 61 (1H),
7. 53-7. 49 (2H), 7. 45-7. 38 (4H), 7. 34-7. 18 (13H),
7. 09-7. 03 (3H), 6. 65 (1H), 6. 20 (1H)_{∘}

### [Example 15]

### <Synthesis of 9-(9H-carbazol-9-yl)-N-{4-(5,8-diphenylnaphthalen-2-yl)phenyl}-N-phenyldibenzo[b,d]furan-3-amine (Compound: B-264)>

To a nitrogen-purged reaction vessel,
3.3 g of 4-(5,8-diphenylnaphthalen-2-yl)-N-phenylaniline, 4.0 g of 9-(7-chlorodibenzo[b,d]furan-1-yl)-9H-carbazole, 0.2 g of tris(dibenzylideneacetone)palladium(0), 0.1 g of tri-tert-butylphosphine, and 1.7 g of sodium tert-butoxy were added, and the mixture was refluxed and stirred overnight under a xylene solvent.

After checking the completion of the reaction, methanol was added, and the precipitated solid was filtered. The obtained crude product was subjected to crystallization and purification using a tetrahydrofuran/ethyl acetate mixed solvent to obtain 4.0 g of a white powder of 9-(9H-carbazol-9-yl)-N-{4-(5,8-diphenylnaphthalen-2-yl)phenyl}-N-phenyldibenzo[b,d]furan-3-amine (Compound: B-264) (yield: 57.5%).

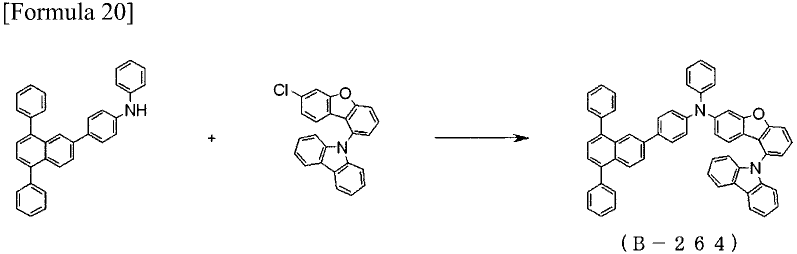

### [Example 16]

### <Synthesis of 9-(9H-carbazol-9-yl)-N-phenyl-N-{4-(7-phenylnaphthalen-1-yl)phenyl}dibenzo[b,d]furan-3-amine (Compound: B-267)>

To a nitrogen-purged reaction vessel,
8.1 g of 9-(9H-carbazol-9-yl)-N-phenyldibenzo[b,d]furan-3-amine, 6.0 g of 1-(4-chlorophenyl)-7-phenylnaphthalene, 0.4 g of tris(dibenzylideneacetone)palladium(0), 0.2 g of tri-tert-butylphosphine, and 3.7 g of sodium tert-butoxy were added, and the mixture was refluxed and stirred overnight under a xylene solvent.

After checking the completion of the reaction, methanol was added, and the precipitated solid was filtered. The obtained crude product was subjected to crystallization and purification using a toluene/methanol mixed solvent to obtain 6.5 g of a white powder of 9-(9H-carbazol-9-yl)-N-phenyl-N-{4-(7-phenylnaphthalen-1-yl)phenyl}dibenzo[b,d]furan-3-amine (Compound: B-267) (yield: 48.5%).

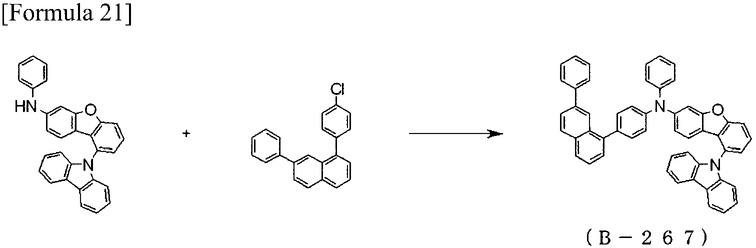

The structure of the obtained white powder was identified using NMR.

The following 34 hydrogen signals were detected by ¹H-NMR (CDCl₃).
δ (ppm) =8. 25 (2H), 8. 20 (1H), 8. 00 (1H), 7. 8 9 (1H),
7. 7 9 (1H), 7. 7 1 (1H), 7. 66 (2H), 7. 6 1 (1H), 7. 5 6 - 7.
46 (5H), 7. 43-7. 38 (5H), 7. 3 7 - 7. 29 (5H), 7. 2 2 - 7.
19 (6H), 7. 09 (1H), 6. 75 (1H), 6. 28 (1H)_{∘}

### [Example 17]

For the arylamine compounds represented by the general formula (1), the melting points and glass transition points were measured using a high-sensitivity differential scanning calorimeter (DSC3100SA manufactured by Bruker AXS). Table 1 summarizes and shows the measurement results.

**[Table 1]**

| | Compound | Melting point [°C] | Glass transition point [°C] |
|---|---|---|---|
| Example 1 | Compound A-1 | - | 110 |
| Example 2 | Compound A-5 | - | 110 |
| Example 3 | Compound A-36 | 246 | 123 |
| Example 4 | Compound B-1 | - | 133 |
| Example 5 | Compound B-40 | - | 144 |
| Example 6 | Compound B-86 | - | 143 |
| Example 7 | Compound A-236 | - | 125 |
| Example 8 | Compound B-6 | - | 144 |
| Example 9 | Compound B-11 | - | 148 |
| Example 10 | Compound B-31 | - | 142 |
| Example 11 | Compound B-146 | - | 133 |
| Example 12 | Compound B-237 | - | 144 |
| Example 13 | Compound B-262 | - | 135 |
| Example 14 | Compound B-263 | - | 111 |
| Example 15 | Compound B-264 | - | 152 |
| Example 16 | Compound B-267 | - | 128 |

Since the arylamine compounds represented by the general formula (1) have glass transition points of 100°C or higher, it is indicated that the thin film states are stable.

### [Example 18]

Vapor-deposited films with a film thickness of 100 nm were prepared on an ITO substrate by using the arylamine compounds represented by the general formula (1), and the work functions (unit: eV) were measured by an ionization potential measuring device (PYS-202, manufactured by Sumitomo Heavy Industries, Ltd.). Table 2 summarizes and shows the measurement results.

**[Table 2]**

| | Compound | Work function [eV] |
|---|---|---|
| Example 1 | Compound A-1 | 5.63 |
| Example 2 | Compound A-5 | 5.63 |
| Example 3 | Compound A-36 | 5.60 |
| Example 4 | Compound B-1 | 5.68 |
| Example 5 | Compound B-40 | 5.68 |
| Example 6 | Compound B-86 | 5.69 |
| Example 7 | Compound A-236 | 5.64 |
| Example 8 | Compound B-6 | 5.71 |
| Example 9 | Compound B-11 | 5.69 |
| Example 10 | Compound B-31 | 5.71 |
| Example 11 | Compound B-146 | 5.71 |
| Example 12 | Compound B-237 | 5.66 |
| Example 13 | Compound B-262 | 5.66 |
| Example 14 | Compound B-263 | 5.72 |
| Example 15 | Compound B-264 | 5.78 |
| Example 16 | Compound B-267 | 5.70 |

Since the arylamine compounds represented by the general formula (1) show preferable energy levels compared to the work function of 5.4 eV of a general hole transport material such as NPD, TPD, or the like, it is understood that they have good hole transport abilities and excellent electron blocking abilities.

### [Example 19]

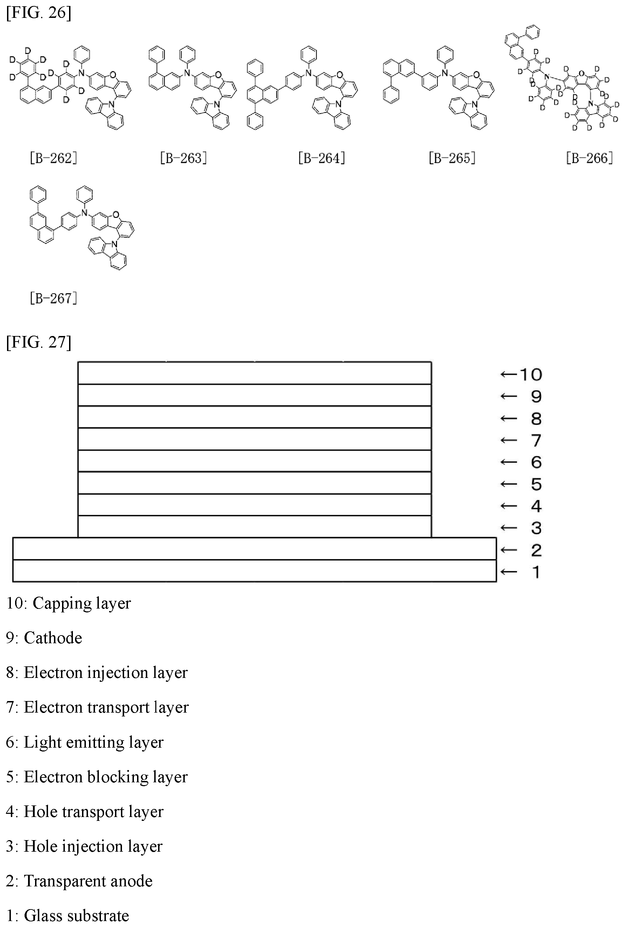

As shown in FIG. 27, the organic EL element was manufactured by depositing a hole injection layer 3, a hole transport layer 4, an electron blocking layer 5, a light emitting layer 6, an electron transport layer 7, an electron injection layer 8, a cathode 9, and a capping layer 10 in that order on a glass substrate 1 on which a reflective ITO electrode had previously been formed as a transparent anode 2.

Specifically, after a glass substrate 1 on which ITO with a film thickness of 50 nm, a silver alloy reflective film with a film thickness of 100 nm, and ITO with a film thickness of 5 nm were sequentially formed as a transparent anode 2 was ultrasonic cleaned in isopropyl alcohol for 20 minutes, drying was performed for 10 minutes on a hot plate heated to 250°C. After that, UV ozone treatment was performed for 2 minutes, and then this glass substrate on which ITO was formed was mounted in a vacuum evaporator, and the pressure was reduced to 0.001 Pa or less.

Subsequently, an electron acceptor (Acceptor-1) of the following structural formula and a compound (HTM-1) of the following structural formula as a hole injection layer 3 to cover the transparent anode 2 were subjected to binary deposition at a deposition rate in which a deposition rate ratio becomes Acceptor-1:HTM-1 = 3:97, to have a film thickness of 10 nm.

On this hole injection layer 3, the compound (HTM-1) of the following structural formula was formed as a hole transport layer 4 to have a film thickness of 140 nm.

On this hole transport layer 4, the compound (A-1) of Example 1 was formed as an electron blocking layer 5 to have a film thickness of 5 nm.

On this electron blocking layer 5, a compound (EMD-1) of the following structural formula and a compound (EMH-1) of the following structural formula as the light emitting layer 6 were subjected to binary deposition at a deposition rate in which a deposition rate ratio becomes EMD-1:EMH-1 = 5:95, to have a film thickness of 20 nm.

On this light emitting layer 6, a compound (ETM-1) of the following structural formula and a compound (ETM-2) of the following structural formula as the electron transport layer 7 were subjected to binary deposition at a deposition rate in which a deposition rate ratio becomes ETM-1:ETM-2 = 50:50, to have a film thickness of 30 nm.

On this electron transport layer 7, lithium fluoride was formed as an electron injection layer 8 to have a film thickness of 1 nm.

On this electron injection layer 8, a magnesium silver alloy was formed as a cathode 9 to have a film thickness of 12 nm.

Finally, a compound (CPL-1) of the following structural formula was formed as the capping layer 10 to have a film thickness of 60 nm.

The properties of the manufactured organic EL element were measured in the atmosphere and at room temperature.

Table 3 summarizes and shows the measurement results of the luminescence characteristics obtained by applying a direct current voltage to the manufactured organic EL element.

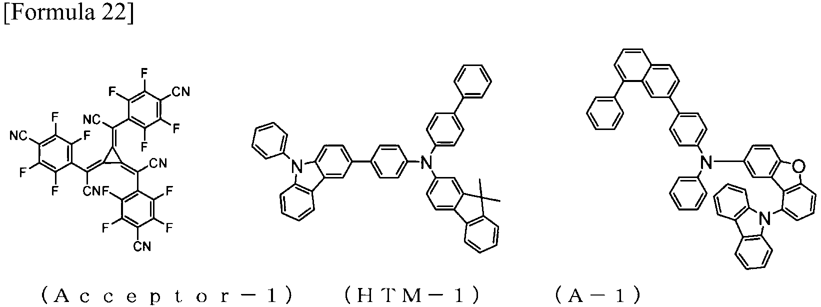

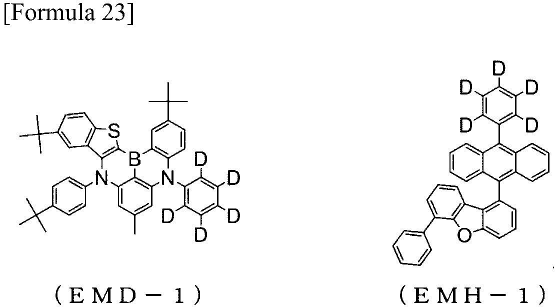

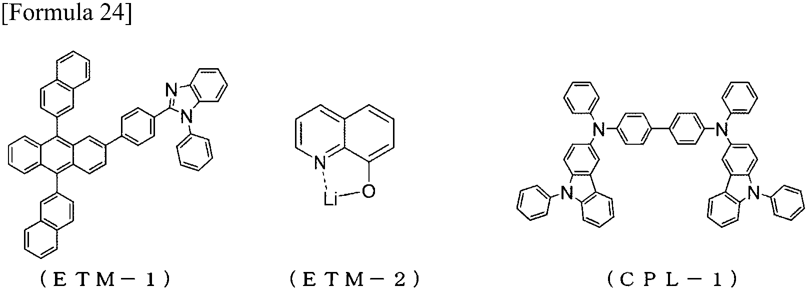

### [Examples 20 to 34]

Organic EL elements were manufactured under the same conditions except that the compounds obtained in Examples 2 to 16, respectively, were used instead of the compound (A-1) in Example 1 as the material of the electron blocking layer 5 in Example 19. The properties of the manufactured organic EL elements were measured in the atmosphere and at room temperature. Table 3 summarizes and shows the measurement results of the luminescence characteristics obtained by applying a direct current voltage to the manufactured organic EL elements.

### [Comparative Example 1]

For comparison, an organic EL element was manufactured under the same conditions except that a compound (HTM-2) (see, e.g., Patent Document 7) of the following structural formula was used instead of the compound (A-1) in Example 1 as the material of the electron blocking layer 5 in Example 19. The properties of the manufactured organic EL element were measured in the atmosphere and at room temperature. Table 3 summarizes and shows the measurement results of the luminescence characteristics obtained by applying a direct current voltage to the manufactured organic EL element.

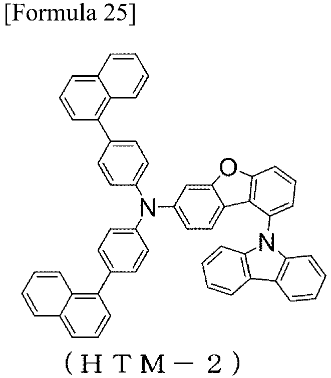

### [Comparative Example 2]

For comparison, an organic EL element was manufactured under the same conditions except that a compound (HTM-3) (see, e.g., Patent Document 8) of the following structural formula was used instead of the compound (A-1) in Example 1 as the material of the electron blocking layer 5 in Example 19. The properties of the manufactured organic EL element were measured in the atmosphere and at room temperature. Table 3 summarizes and shows the measurement results of the luminescence characteristics obtained by applying a direct current voltage to the manufactured organic EL element.

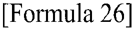

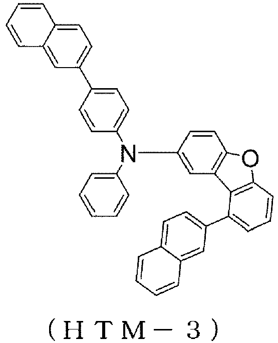

Lifespan of the organic EL elements manufactured in Examples 19 to 34 and Comparative Examples 1 and 2 was measured. The results are summarized and shown in Table 3. When constant current driving was performed with the emission luminance at the start of emission (initial luminance) being 1000 cd/m², the element lifespans were measured as the time until the emission luminance attenuates to 950 cd/m² (equivalent to 95% when the initial luminance is 100%: 95% attenuation).

**[Table 3]**

| | Electron blocking layer | Voltage [V] (@10 mA/cm²) | Luminance [cd/m²] (@10 mA/cm²) | Luminous efficiency [cd/A] (@10 mA/cm²) | Power efficiency [1m/W] (@10 mA/cm²) | Element lifespan 95% attenuation |
|---|---|---|---|---|---|---|
| Example 19 | Compound A-1 | 3.44 | 920 | 9.21 | 8.41 | 583 hours |
| Example 20 | Compound A-5 | 3.43 | 906 | 9.07 | 8.31 | 556 hours |
| Example 21 | Compound A-36 | 3.41 | 907 | 9.07 | 8.36 | 615 hours |
| Example 22 | Compound B-1 | 3.34 | 951 | 9.52 | 8.96 | 658 hours |
| Example 23 | Compound B-40 | 3.33 | 900 | 9.02 | 8.51 | 597 hours |
| Example 24 | Compound B-86 | 3.30 | 890 | 8.95 | 8.45 | 583 hours |
| Example 25 | Compound A-236 | 3.35 | 909 | 9.10 | 9.76 | 595 hours |
| Example 26 | Compound B-6 | 3.36 | 912 | 9.12 | 9.71 | 568 hours |
| Example 27 | Compound B-11 | 3.42 | 923 | 9.23 | 9.11 | 639 hours |
| Example 28 | Compound B-31 | 3.42 | 933 | 9.33 | 9.49 | 586 hours |
| Example 29 | Compound B-146 | 3.39 | 928 | 9.29 | 9.51 | 587 hours |
| Example 30 | Compound B-237 | 3.31 | 920 | 9.20 | 8.91 | 556 hours |
| Example 31 | Compound B-262 | 3.42 | 911 | 9.10 | 9.77 | 637 hours |
| Example 32 | Compound B-263 | 3.36 | 918 | 9.18 | 9.51 | 584 hours |
| Example 33 | Compound B-264 | 3.44 | 932 | 9.33 | 8.96 | 558 hours |
| Example 34 | Compound B-267 | 3.30 | 942 | 9.43 | 8.73 | 590 hours |
| Comparative Example 1 | HTM-2 | 3.50 | 835 | 8.34 | 7.83 | 398 hours |
| Comparative Example 2 | HTM-3 | 3.51 | 825 | 8.25 | 7.52 | 455 hours |

As shown in Table 3, the luminous efficiencies when a current with a current density of 10 mA/cm² is applied had efficiencies of 8.95 to 9.52 cd/A for the organic EL elements of Examples 19 to 34 compared to 8.25 to 8.34 cd/A for the organic EL elements of Comparative Examples 1 and 2. Also, regarding power efficiency, the organic EL elements of Examples 19 to 34 had high efficiencies of 8.31 to 8.96 lm/W compared to 7.52 to 7.83 lm/W for the organic EL elements of Comparative Examples 1 and 2. In addition, it is understood that the element lifespans (95% attenuation) of the organic EL elements of Examples 19 to 34 are increased to 556 to 658 hours compared to 398 to 455 hours for the organic EL elements of Comparative Examples 1 and 2.

As is clear from the above results, since the organic EL element of the present disclosure uses an arylamine compound with high hole mobility and excellent electron blocking ability, it could be found an organic EL element having high luminous efficiency and long lifespan compared to the conventional organic EL element can be realized.

### [INDUSTRIAL APPLICABILITY]

The organic EL element using the arylamine compound having the specific structure of the present disclosure can improve the durability of the organic EL element along with improved luminous efficiency, and for example, it has become possible to deploy it in home telephone products and lighting applications.

### [EXPLANATION OF REFERENCE NUMERALS]

1: Glass substrate
2: Transparent anode
3: Hole injection layer
4: Hole transport layer
5: Electron blocking layer
6: Light emitting layer
7: Electron transport layer
8: Electron injection layer
9: Cathode
10: Capping layer

## Claims

1. An arylamine compound represented by the general formula (1) below: 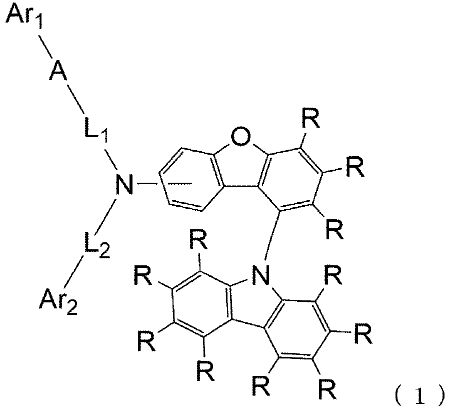
wherein, in the formula, A indicates a substituted or unsubstituted naphthylene group, L₁ and L₂ indicate a single bond, a substituted or unsubstituted divalent aryl group, or a substituted or unsubstituted divalent heteroaryl group,
R is identical to or different from each other, and indicates a hydrogen atom, a deuterium atom, a substituted or unsubstituted aryl group, or a substituted or unsubstituted heteroaryl group, and
Ar₁ and Ar₂ indicate a substituted or unsubstituted aryl group, or a substituted or unsubstituted heteroaryl group.

2. The arylamine compound according to claim 1,
wherein the general formula (1) is represented by the general formula (1-1) or general formula (1-2) below: 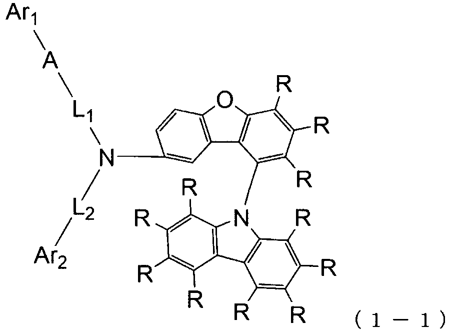 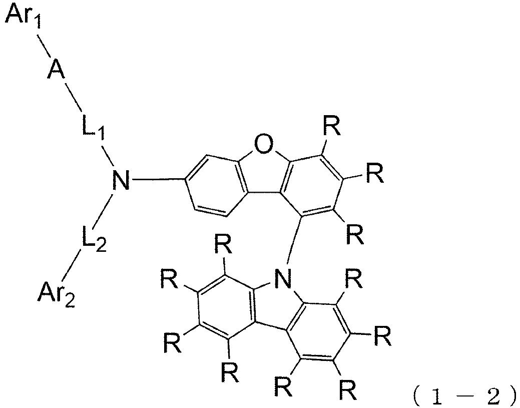
wherein, in the formulas, A, Ar₁, Ar₂, L₁, L₂ and R are as defined in the general formula (1).

3. The arylamine compound according to claim 2,
wherein A in the general formula (1-1) or general formula (1-2) is represented by the general formula (2-1) to general formula (2-6) below: 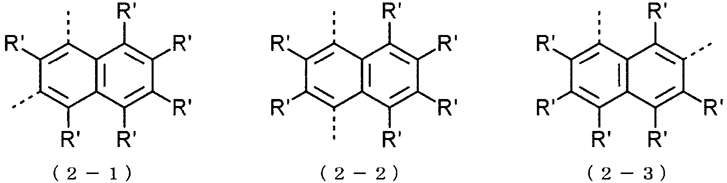 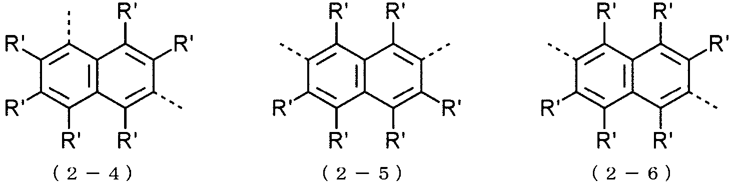
wherein, in the formulas, a dashed line indicates a bonding position to Ar₁ or L₁, and
R' is identical to or different from each other, and indicates a hydrogen atom, a deuterium atom, an unsubstituted phenyl group, an unsubstituted biphenylyl group, an unsubstituted naphthyl group, or an unsubstituted phenanthryl group.

4. The arylamine compound according to claim 3,
wherein L₁ and L₂ in the general formula (1-1) or (1-2) indicate a single bond, a substituted or unsubstituted divalent phenylene group, a substituted or unsubstituted divalent naphthylene group, or a substituted or unsubstituted divalent biphenylylene group.

5. The arylamine compound according to claim 3,
wherein R in the general formula (1-1) or general formula (1-2) indicates a hydrogen atom, a deuterium atom, a substituted or unsubstituted phenyl group, or a substituted or unsubstituted naphthyl group.

6. The arylamine compound according to claim 3,
wherein Ar₁ in the general formula (1-1) or general formula (1-2) indicates a substituted or unsubstituted phenyl group, a substituted or unsubstituted biphenylyl group, a substituted or unsubstituted naphthyl group, a substituted or unsubstituted dibenzofuranyl group, or a substituted or unsubstituted phenanthryl group.

7. The arylamine compound according to claim 6,
wherein Ar₁ in the general formula (1-1) or general formula (1-2) indicates an unsubstituted phenyl group, an unsubstituted biphenylyl group, an unsubstituted naphthyl group, an unsubstituted dibenzofuranyl group, or an unsubstituted phenanthryl group.

8. An organic EL element having a pair of electrodes and at least one organic layer sandwiched between them,
wherein the organic layer contains the arylamine compound described in claim 1 or 2.

9. The organic EL element according to claim 8,
wherein the organic layer is a hole transport layer, an electron blocking layer, a hole injection layer, or a light emitting layer.

10. An electronic device having a pair of electrodes and at least one organic layer sandwiched between them,
wherein the organic layer contains the arylamine compound described in claim 1 or 2.
